# FASCICULE DE BREVET EUROPEEN

(11) **EP 1 101 765 B1**
(45) Date de publication et mention de la délivrance du brevet: **14.08.2002**
(21) Numéro de dépôt: 00403108.4
(22) Date de dépôt: 09.11.2000
(51) Int. Cl.: C07D 471/14, A61K 31/4375, A61P 35/00, C07D 491/22

(54) **Nouveaux composés analogues de la camptothécine, leur procédé de préparation et les compositions pharmaceutiques qui les contiennent**
Camptothecin Analoge Verbindungen, Verfahren zu ihrer Herstellung und diese enthaltende pharmazeutische Zubereitungen
Camptothecin analogues, process for their preparation and pharmaceutical compositions containing them

(30) Priorité: 18.11.1999 FR 9914499
(43) Date de publication de la demande: 23.05.2001
(73) Titulaire: LES LABORATOIRES SERVIER, 92200 Neuilly sur Seine (FR)
(72) Inventeur: Lavielle, Gilbert, 78170 La Celle Saint Cloud (FR); Hautefaye, Patrick, 77170 Servon Brie Comte Robert (FR); Pierre, Alain, 78580 Les Alluets le Roi (FR); Atassi, Ghanem, 92210 Saint Cloud (FR); Hickman, John, 92800 Puteaux (FR); Cimetiere, Bernard, 75020 Paris (FR)

(56) Documents cités:
- WO-A-98/28304
- RIGBY J H ET AL: "Vinyl Isocyanates in Alkaloid Synthesis. Camptothecin Model Studies" TETRAHEDRON LETTERS,NL,ELSEVIER SCIENCE PUBLISHERS, AMSTERDAM, vol. 38, no. 28, 14 juillet 1997 (1997-07-14), pages 4969-4972, XP004081271 ISSN: 0040-4039

## Description

La présente invention concerne de nouveaux composés analogues de la camptothécine, leur procédé de préparation et les compositions pharmaceutiques qui les contiennent.

La camptothécine, alcaloïde isolé de *Camptothéca accuminata*, est un agent anticancéreux possédant un large spectre d'activité. Son caractère insoluble a longtemps orienté les recherches vers les sels solubles de ce composé, dont la toxicité s'est révélé être un handicap majeur. Plusieurs autres études ont été menées en vue d'obtenir des analogues structuraux et de palier le manque de solubilité de la molécule naturelle (J. Med. Chem., 1991, 34, 98 ; Chem. Pharm. Bull., 1991, 39, 3183). Les modifications apportées concernent principalement les cycles A et B. D'autre part les conclusions de différentes études montrent l'importance du cycle E et plus particulièrement de la fonction lactone. En effet, cette dernière, en équilibre avec la forme ouverte hydroxy-acide, semble être la forme la plus active et possédant des effets indésirables réduits (Cancer Res., 1989, 49, 1465 ; ibid, 1989, 49, 5077). Des tentatives de modification de ce cyle ont été menées, en particulier l'atome d'oxygène cyclique a été remplacé par un atome d'azote ou de soufre, mais dans chaque cas il y a perte de l'activité pharmacologique confirmant ainsi l'importance de la lactone (J. Med. Chem., 1989, 32, 715).

La présente invention concerne des composés analogues structuraux de la camptothécine dont le cycle E a été modifié. Ils se caractérisent par le remplacement de la fonction lactone de ce cycle par une fonction cétone cyclique, l'oxygène cyclique ayant été remplacé par un atome de carbone. Les composés ainsi obtenus possèdent une structure originale et présentent de façon surprenante un caractère cytotoxique important. Ils pourront donc être utilisés pour la fabrication de médicaments utiles dans le traitement des maladies cancéreuses.

L'invention concerne les composés de formule (I) : 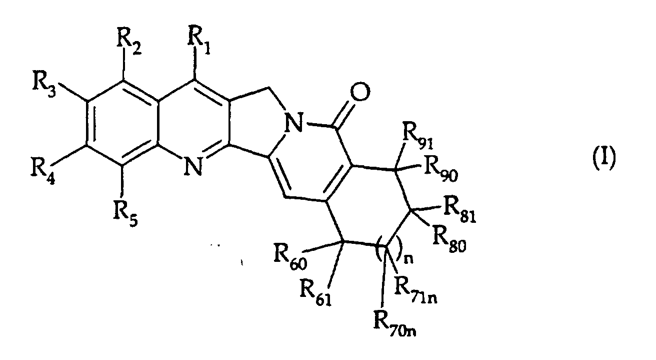 dans laquelle :
- n vaut 0, 1 ou 2,
- R₁, R₂, R₃, R₄ et R₅ sont choisis indépendamment parmi un atome d'hydrogène, d'halogène, un groupement alkyle, alkényle, alkynyle, perhalogénoalkyle, cycloalkyle(C₃-C₁₁), (C₃-C₁₁) cycloalkylalkyle, hydroxy, hydroxyalkyle, alkoxy, alkoxyalkyle, alkoxycarbonyle, acyloxy, carboxy, nitro, cyano, aminocarbonyle (éventuellement substitué sur l'atome d'azote par un ou deux groupements alkyle), et les groupements (CH₂)ₚ-NRₐR_{b}, et -O-C(O)-N-RₐR_{b}, dans lesquels p est un entier compris entre 0 et 6, et Rₐ et R_{b} représentent indépendamment un atome d'hydrogène, un groupement alkyle, cycloalkyle (C₃-C₁₁), (C₃-C₁₁) cycloalkylalkyle, acyle, aryle éventuellement substitué, arylalkyle éventuellement substitué, ou bien Ra et Rb forment ensemble avec l'atome d'azote qui les porte un groupement pyrrolyle, pipéridinyle, ou pipérazinyle, chacun de ces groupements cycliques pouvant être éventuellement substitué,
ou bien deux groupements R₂, R₃, R₄ et R₅ adjacents forment ensemble avec les atomes de carbone qui les portent un groupement -O-(CH₂)ₜ-O, t étant un entier compris entre 1 et 3 inclus,
- R₆₀, R₇₀ₙ, R₈₀ et R₉₀ représentent indépendamment un atome d'hydrogène, un groupement hydroxy, alkoxy, ou un groupement O-(CO)-X ou O-(CO)-NXW dans lesquels X et W représentent indépendamment un groupement alkyle, alkényle, alkynyle, cycloalkyle (C₃-C₁₁), (C₃-C₁₁) cycloalkylalkyle, aryle éventuellement substitué, ou arylalkyle éventuellement substitué,
- R₆₁, R₇₁ₙ, R₈₁ et R₉₁ représentent indépendamment un atome d'hydrogène, un groupement alkyle, alkényle, alkynyle, ou, pris deux à deux sur des carbones adjacents, forment ensemble une liaison, ou un groupement oxirane, ou bien deux groupements géminaux (R₆₀ et R₆₁) et/ou (R₇₀ₙ et R₇₁ₙ) et/ou (R₈₀ et R₈₁) et/ou (R₉₀ et R₉₁) forment ensemble un groupement oxo ou un groupement -O-(CH₂)ₜ₁-O, t₁ étant un entier compris entre 1 et 3 inclus,
à la condition que R₆₀, R₆₁, R₇₀ₙ, R₇₁ₙ, R₈₀, R₈₁, R₉₀, R₉₁ ne représentent pas tous un atome d'hydrogène,
leurs énantiomères, diastéréoisomères, ainsi que leurs sels d'addition à un acide ou à une base pharmaceutiquement acceptable,
étant entendu que
- le terme alkyle désigne une chaîne de 1 à 6 atomes de carbone, linéaire ou ramifié,
- le terme alkényle désigne une chaîne de 2 à 6 atomes de carbone, linéaire ou ramifié et contenant de 1 à 3 doubles liaisons,
- le terme alkynyle désigne une chaîne de 2 à 6 atomes de carbone, linaire ou ramifié et contenant de 1 à 3 triples liaisons,
- le terme alkoxy désigne un radical alkyle-oxy linéaire ou ramifié contenant de 1 à 6 atomes de carbone,
- le terme acyle désigne un radical alkyle-carbonyle linéaire ou ramifié contenant de 1 à 6 atomes de carbone,
- le terme aryle représente un groupement phényle ou naphtyle,
- l'expression "substitué" lorsqu'elle concerne les groupements aryle ou arylalkyle signifie que les groupements concernés sont substitués par un ou plusieurs atomes d'halogène, et/ou groupement alkyles, alkoxy, hydroxy, cyano, nitro, et/ou amino (éventuellement substitué par un ou deux groupements alkyle),
- l'expression "substitué" lorsqu'elle concerne les groupements pyrrolyle, pipéridinyle, ou pipérazinyle signifie que les groupements concernés sont substitués par un ou plusieurs groupements alkyle, alkoxy, aryle, arylalkyle, aryloxy, et/ou aryloxyalkyle.

Un aspect avantageux de l'invention concerne les composés de formule (I) pour lesquels R₆₀ représente un groupement hydroxy et R₆₁ représente un groupement alkyle (par exemple éthyle).

Un autre aspect avantageux de l'invention concerne les composés de formule (I) pour lesquels R₈₀ et R₈₁ forment ensemble un groupement oxo, ou bien R₉₀ et R₉₁ forment ensemble un groupement oxo, ou bien R₈₀ et R₈₁ ainsi que R₉₀ et R₉₁ forment deux groupements oxo.

Des composés préférés de formule (I) sont ceux pour lesquels R₁ représente un atome d'hydrogène.

D'autres composés préférés de formule (I) sont ceux pour lesquels R₂, R₃, R₄ et R₅ sont choisis parmi un atome d'hydrogène, d'halogène, un groupement alkyle, alkoxy, ou bien deux de ces groupements lorsqu'ils sont reliés à deux carbones adjacents forment ensemble un groupement méthylènedioxy ou éthylènedioxy (préférentiellement méthylènedioxy). Parmi ces composés, on préférera ceux pour lesquels R₂ et R₅ représentent chacun un atome d'hydrogène.

Un aspect particulièrement avantageux de l'invention concerne les composés de formule (I) pour lesquels R₁, R₂ et R₅ représentent chacun un atome d'hydrogène, R₃ et R₄ sont choisis parmi un atome d'hydrogène, d'halogène, un groupement alkyle, alkoxy, ou bien forment ensemble un groupement méthylènedioxy, R₆₀, R₇₀ₙ, R₈₀ et R₉₀ représentent indépendamment un atome d'hydrogène, un groupement hydroxy, ou alkoxy, et R₆₁, R₇₁ₙ, R₈₁ et R₉₁ représentent indépendamment un atome d'hydrogène, un groupement alkyle, ou pris deux à deux sur des carbones adjacents forment ensemble une liaison ou un groupement oxirane, ou bien deux groupements géminaux (R₆₀ et R₆₁) et/ou (R₇₀ₙ et R₇₁ₙ) et/ou (R₈₀ et R₈₁) et/ou (R₉₀ et R₉₁) forment ensemble un groupement oxo.

La présente invention concerne également le procédé de préparation des composés de formule (I), caractérisé en ce que l'on utilise comme produit de départ un composé de formule (II) : 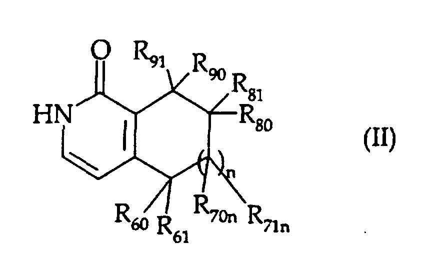 dans laquelle n, R₆₀, R₆₁, R₇₀ₙ, R₇₁ₙ, R₈₀, R₈₁, R₉₀, R₉₁ sont tels que définis dans la formule (I),
qui est condensé, en milieu basique, sur un composé de formule (III) : 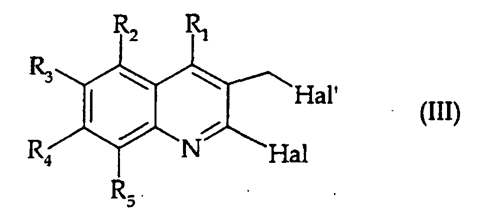 dans laquelle R₁, R₂, R₃, R₄, et R₅ sont tels que définis dans la formule (I), et Hal et Hal' représentent indépendamment un atome d'halogène,
ou bien, selon les conditions de réaction de Mitsunobu, sur un composé de formule (III') : 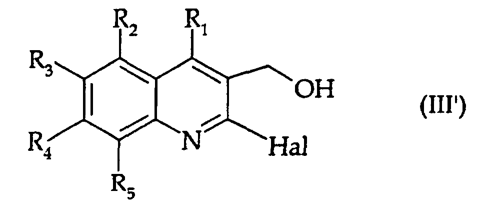 dans laquelle R₁, R₂, R₃, R₄, R₅ et Hal sont tels que définis précédemment,
pour conduire à un composé de formule (IV) : 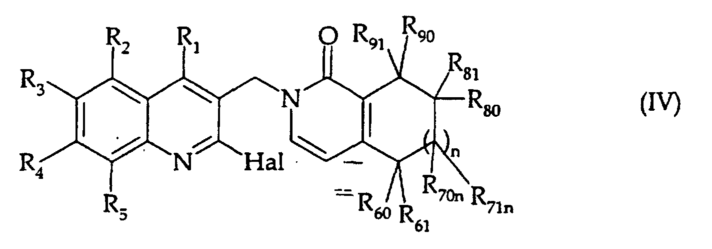 dans laquelle R₁, R₂, R₃, R₄, R₅, n, R₆₀, R₆₁, R₇₀ₙ, R₇₁ₙ, R₈₀, R₈₁, R₉₀, R₉₁ et Hal sont tels que définis précédemment,
composé (IV) qui subit une réaction de cyclisation intramoléculaire de type "Heck" catalysé par un dérivé du palladium, pour conduire au composé de formule (I),
étant entendu, dans le but de simplifier le procédé ci-dessus, que les groupements réactifs présents en R₆₀, R₆₁, R₇₀ₙ, R₇₁ₙ, R₈₀, R₈₁, R₉₀, R₉₁ peuvent être protégés à l'aide de groupements protecteurs classiques et déprotégés au moment opportun, que les groupements hydroxy présents en ces mêmes positions peuvent être oxydés par des méthodes classiques de la chimie, en groupement oxo, que inversement les groupements oxo présents en ces mêmes positions peuvent être réduits par des réducteurs classiques, à tout moment opportun de la synthèse, et que lorsque deux de ces groupements forment ensemble une liaison, cette dernière peut être introduite à tout moment jugé utile par l'homme de métier afin de faciliter la synthèse,
composés de formule (I) :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

Les substrats de formule (II) et (III) sont commerciaux ou préparés selon des modes opératoires connus ou en utilisant des réactions classiques de la chimie organique. A titre d'illustration on peut citer les procédés décrits dans J. Am. Chem. Soc., 1992, 37, 10971.

En particulier, il est envisageable de préparer un substrat de formule (II') : 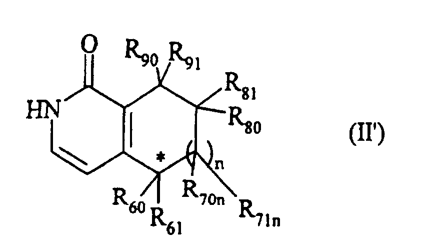 dans laquelle n, R₆₀, R₆₁, R₇₀ₙ, R₇₁ₙ, R₈₀, R₈₁, R₉₀ et R₉₁ sont tels que définis dans la formule (I) et * indique que le carbone portant les groupements R₆₀ et R₆₁ possède une configuration fixée (R) ou (S),
afin d'obtenir selon le procédé précédemment décrit, un composé de formule (I') : 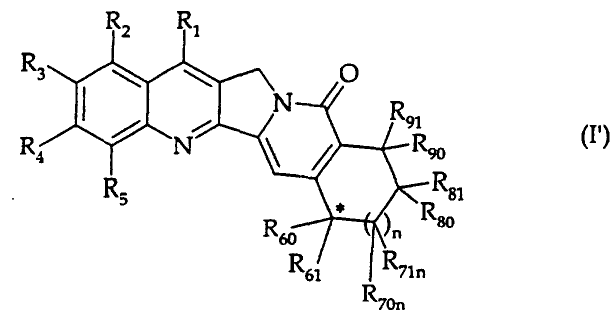 dans laquelle R₁, R₂, R₃, R₄, R₅, n, R₆₀, R₆₁, R₇₀ₙ, R₇₁ₙ, R₈₀, R₈₁, R₉₀ et R₉₁ sont tels que définis dans la formule (I) et * indique que la configuration du carbone est fixée.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

Les structures des composés décrits dans les exemples et les préparations ont été déterminées selon les techniques spectroprotométriques usuelles (infrarouge, RMN, spectrométrie de masse, ...).

### PREPARATION A : 7-Hydroxy-6,7-dihydro-1H-cyclopenta[c]pyridine-1,5(2H)-dione

### Stade 1 : 4-(1-Ethoxyvinyl)-2-fluoro-3-pyridinecarbaldehyde

On mélange succesivement 8 g (31,9 mmol) de 2-fluoro-4-iodo-3-pyridinecarbaldéhyde (décrit dans J.O.C, 1993, 58, pp. 7832), 12,7 g (35,96 mmol) de 1-éthoxyvinyltributylétain, 740 mg de tétrakistriphénylphosphinepalladium (2 % molaire) dans 280 mL de dioxane. La réaction est chauffée à reflux jusqu'à disparition complète du produit de départ. Après retour à la température ambiante, une solution de fluorure de potassium est ajoutée au mélange réactionnel. Une agitation vigoureuse est maintenue pendant 10 minutes afin de faire précipiter le fluorure de tributylétain que l'on filtre. Le filtrat est plongé dans une solution de chlorure de sodium. La phase aqueuse est extraite 3 fois avec du dichlorométhane puis les phases organiques sont regroupées, séchées sur sulfate de magnésium, concentrées et purifiées par chromatographie sur colonne de silice, en utilisant comme éluant un mélange dichlorométhane/cyclohexane (95/5) pour conduire au produit attendu.

### Stade 2 : 7-Hydroxy-6,7-dihydro-1H-cyclopenta[c]pyridine-1,5(2H)-dione

Un mélange 770 g (3,93 mmol) du composé décrit au stade précédent dans 51 mL (102,1 mmol) d'acide chlorhydrique 2N est porté à reflux à l'air pendant 2 heures, refroidi puis concentré. Le résidu obtenu est lavé dans l'acétone, filtré puis recristallisé dans l'isopropanol pour obtenir le produit attendu

### Point de fusion : 200°C (isopropanol)

### PREPARATION B : 5-Ethyl-5,7-dihydroxy-2,5,6,7-tétrahydro-1H-cyclopenta[c] pyridin-1-one

### Stade 1 : 1-Fluoro-7-hydroxy-6,7-dihydro-5H-cyclopenta[c]pyridine-5-one

Le procédé utilisé est identique à celui décrit dans la préparation A, stade 1, en utilisant un mélange dichlorométhane/méthanol comme éluant au cours de la purification. Le résidu obtenu est agité à température ambiante pendant 3 heures dans une solution de 16 mL d'acide trifluoroacétique, 90 mL d'acétonitrile et 35 mL d'eau. Le milieu réactionnel est ensuite neutralisé avec une solution aqueuse de K₂CO₃ et extrait 3 fois au dichlorométhane. Les phases organiques réunies sont séchées sur sulfate de magnésium, filtrées, concentrées et purifiées par chromatographie sur colonne de silice (éluant : dichlorométhane/méthanol 99,5/0,5) pour obtenir le composé attendu.

### Stade 2 : 5,7-Dihydroxy-5-éthyl-1-fluoro-6,7-dihydro-1H-cyclopenta[c]pyridine

A -30°C, sont ajoutées goutte à goutte 1,6 mL (4,7 mmol) d'une solution de bromure d'éthylmagnésium 3M dans l'éther à une solution de 350 mg (2,1 mmol) de composé décrit au stade précédent dans 60 mL d'éther. Le milieu réactionnel est maintenu sous agitation à -30°C pendant 3 heures avant d'être l'hydrolysé à l'aide d'une solution saturée de chlorure d'ammonium. La phase organique est décantée, la phase aqueuse extraite 3 fois avec de l'acétate d'éthyle puis les phase organiques réunies séchées sur sulfate de magnésium sont filtrée, concentrées, et purifiées par chromatographie sur colonne de silice (éluant : dichlorométhane/acétone, 95/5), pour conduire au produit attendu.

### Stade 3 : 5-Ethyl-5,7-dihydroxy-2,5,6,7-tétrahydro-1H-cyclopenta[c]pyridine-1-one

Le produit attendu est obtenu selon le procédé décrit au stade 2 de la préparation A en utilisant comme produit de départ le composé décrit au stade précédent.

### PREPARATION C : 5-Ethyl-5,6-dihydroxy-2,5-dihydro-1H-cyclopenta[c]pyridine-1-one

A une solution de 2 g du produit décrit dans la préparation F (9,65 mmol) dans 20 ml de méthanol, est ajouté à température ambiante 0,5 g de borohydrure de sodium (13.20 mmoles). Après 30 minutes d'agitation à température ambiante, 20 ml d'une solution aqueuse saturée en hydrogènocarbonate de sodium sont ajoutés. Le milieu est extrait à l'acétate d'éthyle. Les phases organiques sont séchées sur sulfate de magnésium, filtrées, concentrées sous vide pour obtenir le composé attendu.

### PREPARATION D : 5-Ethyl-5-méthoxyméthoxy-2H,5H-[2]pyridine-1,6,7-trione

A une solution de 3 g (10,6 mmol) du composé préparé au stade 3 de la préparation F dans 130 ml de dioxane est ajoutée une solution de 2,35 g (21,2 mmol) d'oxyde de sélénium dans 3 ml d'eau. Le mélange réactionnel est chauffé à 60°C pendant 48 heures. Le milieu réactionnel est filtré, le filtrat est concentré sous vide, le résidu est repris dans de l'acétate d'éthyle. Le produit attendu est obtenu par filtration du précipité.

### PREPARATION E : 5-Ethyl-5-hydroxy-5,6-dihydro-1H-cyclopenta[c]pyridine-1,7 (2H)-dione

### Stade 1 : 5-Ethyl-1-fluoro-5-hydroxy-1,2,5,6-tétrahydro-7H-cyclopenta[c]pyridine-7-one

A une solution agitée à 0°C de 3 g (15,2 mmol) du composé décrit au stade 2 de la préparation B dans un mélange de dichlorométhane/acétone (15/1 v/v) sont ajoutés successivement 3 g de célite, du tamis moléculaire 4Å, puis par petites fractions 3,6 g (16,7 mmol) de chlorochromate de pyridinium. Le mélange réactionnel est agité à température ambiante pendant 20 heures, filtré, le solide est lavé par 3 fois 50 mL d'un mélange dichlorométhane/acétone (50/50 v/v), et le filtrat est concentré sous vide. Le résidu obtenu est purifié par chromatographie sur silice (éluant dichlorométhane/acétone 85/15) pour conduire au produit attendu.

### Stade 2 : 5-Ethyl-5-hydroxy-5,6-dihydro-1H-cyclopenta[c]pyridine-1,7(2H)-dione

Une suspension de 3,5 g (17,7 mmol) du composé décrit au stade précédent dans 360 mL d'acide chlorhydrique 1N est chauffée à 80°C pendant 1 heure. Le milieu réactionnel est concentré sous vide. Le résidu solide est agité dans de l'éther, le précipité est filtré et séché pour conduire au produit attendu.

### PREPARATION F : 5-Ethyl-6-[2-(1,3-dioxolan)yl]-5-hydroxy-5,7-dihydro-1H-cyclopenta[c]pyridin-1(2H)-one

### Stade 1 : 2-(2-Fluoro-3-méthyl-4-pyridinyl)-2-hydroxybutanoate de méthyle

A une solution agitée à -78°C de 2,6 g (11 mmol) de 2-fluoro-4-iodo-3-méthylpyridine (décrite dans J.O.C., 1993, 58, pp.7832) dans 100 ml de THF, sont ajoutés goutte à goutte 6,9 ml (11 mmol) de butyllithium 1,6 M dans l'hexane. Le milieu réactionnel est ensuite agité 10 mn à -78°C avant addition d'une solution de 1,5 g (12,9 mmol) de 2-oxo-butyrate de méthyle dans 20 ml de THF. Le milieu réactionnel est agité à 10 mn à -78°C, puis hydrolyé par un mélange eau/THF. La phase aqueuse est décantée puis extraite par 3 fois 50 ml d'acétate de méthyle. Les phases organiques réunies sont séchées, concentrées et purifiées par chromatographie sur silice (éluant : dichlorométhane/acétone 95/5), pour conduire au produit attendu.

### Stade 2 : 2-(2-Fluoro-3-méthyl-4-pyridinyl)-2-méthoxyméthoxylbutanoate de méthyle

A une solution agitée à 0°C de 2 g (8,8 mmol) du composé décrit au stade précédent dans 60 mL de THF sont ajoutés par petites fractions 260 mg (8,8 mmol) d'une suspension d'hydrure de sodium à 80 % dans l'huile minérale. L'addition terminée, le milieu est agité à température ambiante jusqu'à la fin du dégagement gazeux. Une solution de 0,94 g (11.6 mmol) de chlorométhyl-méthyléther dans 10 mL de THF est alors ajoutée goutte à goutte et le milieu est ensuite agité 2h à température ambiante, hydrolysé avec de l'acide chlorhydrique 2N et décanté. La phase aqueuse est extraite par du dichlorométhane, les phases organiques réunies sont séchées sur sulfate de magnésium, et concentrées. Le produit attendu est obtenu par chromatographie sur silice du résidu (éluant dichlorométhane/acétone 95/5).

### Stade 3: 5-Ethyl-1-fluoro-5-(méthoxyméthoxy)-5,7-dihydro-6H-cyclopenta[c] pyridin-6-one

A une solution agitée à 0°C de 9 g (88,5 mmol) de diisopropylamine dans 160 mL de THF sont ajoutés 55 mL (88,5 mmol) d'une solution de butyllithium 1,6 M dans l'hexane. Le milieu est agité 1/2h à 0°C avant d'ajouter goutte à goutte à cette température 15,8 g (88,5 mmol) d'hexaméthylphosphoramide. Le milieu est à nouveau maintenu sous agitation à 0°C 1/2h avant d'ajouter goutte à goutte une solution de 5,3 g (19,5 mmol) du composé décrit au stade précédent dans 40 mL de THF. Le mélange réactionnel est agité 1h à température ambiante avant d'être hydrolysé à l'eau. La phase aqueuse est extraite à l'acétate d'éthyle, la phase organique est lavée à l'aide d'une solution d'acide chlorhydrique 2N, puis par une solution saturée en chlorure de sodium, séchée sur sulfate de magnésium, et concentrée. Le résidu obtenu est purifié par chromatographie sur silice (éluant : dichlorométhane/acétone 95/5) pour conduire au produit attendu.

### Stade 4 : 5-Ethyl-5-hydroxy-5,7-dihydro-1H-cyclopenta[c]pyridine-1,6(2H)-dione

Une suspension de 1 g (4,18 mmol) du composé décrit à l'étape précédente dans 40 ml d'acide chlorhydrique 3N est portée à reflux pendant 1 heure. Le milieu est concentré, le résidu obtenu est repris dans l'éther, décanté et après addition d'acétonitrile, le solide obtenu est filtré, et lavé à l'éther pour conduire au produit attendu.

### Stade 5 : 5-Ethyl-6-[2-(1,3-dioxolan)yl]-5-hydroxy-5,7-dihydro-1H-cyclopenta[c] pyridin-1(2H)-one

Une suspension de 2,5 g (13 mmol) du composé préparé au stade précédent, de 15 ml d'éthylèneglycol, et de 40 mg d'acide paratoluènesulfonique dans 40 ml de toluène est portée au reflux pendant 4 heures. Le milieu est concentré sous vide, le résidu noir est repris dans de l'acétate d'éthyle, le précipité blanc obtenu est filtré pour fournir le produit attendu.

### PREPARATION G : 5-Ethyl-5-hydroxy-7,8-dihydro-1,6(2H,5H)-isoquinolinedione

### Stade 1 : 3-(1,3-Dioxolan-2-yl)-2-fluoro-4-iodopyridine

Dans un tricol équipé d'un Dean Stark, un mélange constitué de 5,1 g (20,3 mmol) de 2-fluoro-4-iodo-3-pyridinecarbaldéhyde, 1,4 g (22,3 mmol) d'éthane-1,2-diol, et de 50 mg d'acide paratoluènesulfonique dans 100 mL de toluène est chauffé à reflux. Lorsque la quantité d'eau théorique a été recueillie, le milieu réactionnel refroidi à l'ambiante est lavé avec une solution saturée de bicarbonate de sodium, et séché sur sulfate de magnésium. Le produit attendu est obtenu après concentration.

### Stade 2 : 2-[3-(1,3-Dioxolan-2-yl)-2-fluoro-4-pyridinyl]-2-hydroxybutanoate de méthyle

A une solution agitée sous atmosphère d'argon à -78°C de 3,1 g (11 mmol) de 3-(1,3-dioxolan-2-yl)-2-fluoro-4-iodopyridine dans 100 mL de THF, sont ajoutés goutte à goutte 6,9 mL (11 mmol) de butyllithium 1,6 M dans l'hexane. Le milieu réactionnel est ensuite agité 10 minutes à -78°C avant addition goutte à goutte d'une solution de 1,5 g (13 mmol) de 2-oxo-butyrate de méthyle dans 20 mL de THF. Le milieu est agité 3 heures à -78°C, hydrolysé par un mélange eau/THF, et décanté. La phase aqueuse est extraite par 3 fois 50 mL d'acétate d'éthyle, les phases organiques réunies sont séchées sur sulfate de magnésium puis concentrées. Le produit attendu est obtenu par purification du résidu par chromatographie sur silice (éluant : dichlorométhane/acétone 95/5).

### Stade 3 : 2-(Benzyloxy)-2-[3-(1,3-dioxolan-2-yl)-2-fluoro-4-pyridinyl]butanoate de méthyle

Une solution de 14,8 g (51,8 mmol) du composé décrit au stade précédent dans 100 ml de DMF est ajoutée goutte à goutte à une suspension agitée à 0°C de 2 g (67,4 mmol) d'hydrure de sodium à 80 % dans 100 ml de DMF. L'addition terminée, le milieu est agité ½ heure avant d'ajouter à cette température successivement 0,15 g d'iodure de tétrabutylammonium, puis 9,8 g (57,1 mmol) de bromure de benzyle en solution dans 50 ml de DMF. Le milieu est agité 2 heures à 0°C, plongé dans de l'eau glacée, extrait à l'acétate d'éthyle, et la phase organique est lavée à l'eau, puis séchée sur sulfate de magnésium et concentrée. Le produit attendu est obtenu par purification du résidu sur gel de silice (éluant : dichlorométhane/acétone 95/5).

### Stade 4: 2-(Benzyloxy)-2-[3-(diméthoxyméthyl)-2-fluoro-4-pyridinyl]butanoate de méthyle

Une solution de 3,4 g (9 mmol) de l'ester décrit au stade précédent et de 0,1 g d'acide paratoluènesulfonique dans 250 mL de méthanol est agitée à température ambiante pendant 24 heures. Le solvant est concentré. Le résidu brut est repris dans l'acétate d'éthyle, la phase organique est neutralisée à l'aide d'une solution saturée de bicarbonate de sodium, lavée avec de l'eau, et séchée sur sulfate de magnésium. Le produit attendu est obtenu après concentration.

### Stade 5 : 2-(Benzyloxy)-2-(2-fluoro-3-formyl-4-pyridinyl)butanoate de méthyle

Une solution de 20,3 g (0,178 mmol) d'acide trifluoroacétique dans 20 mL d'eau est ajoutée à une solution de 3,3 g (8,75 mmol) de l'ester décrit au stade précédent dans 60 mL de dichlorométhane. Le milieu réactionnel est agité 18 heures à température ambiante, et décanté. La phase aqueuse est extraite au dichlorométhane, les phases organiques réunies sont lavées par une solution de bicarbonate de sodium, puis à l'eau salée, et séchées sur sulfate de magnésium. Le produit attendu est obtenu après concentration.

### Stade 6: 3{4-[1-(Benzyloxy)-1-(méthoxycarbonyl)propyl]-2-fluoro-3-pyridinyl}-2-acrylate de méthyle

Un mélange de 21 g (63,3 mmol) du produit décrit au stade précédent et de 22,3 g (63,3 mmol) de carboxyméthylène triphénylphophorane dans 1 litre de toluène est chauffé au reflux pendant 2 heures 30. Après retour à l'ambiante, le solvant est évaporé, le résidu est cristallisé dans l'éther, les cristaux sont filtrés et le filtrat est concentré sous vide. Le résidu est purifié par chromatographie sur gel de silice (éluant : dichlorométhane/acétone, 100/0 à 96/4) pour conduire au produit attendu.

### Stade 7 : 2-(Benzyloxy)-2{2-fluoro-3-[2-(méthoxycarbonyl)éthyl]-4-pyridinyl} butanoate de méthyle

A une solution agitée à 0°C de 16,3 g (42 mmol) du composé décrit au stade précédent dans 900 ml de méthanol sont ajoutés 2,5 g (10,5 mmol) de chlorure de cobalt. Le milieu réactionnel est agité à 0°C pendant 10 minutes puis 3,2 g (84 mmol) de borohydrure de sodium sont ajoutés par petites fractions. Le mélange est ensuite concentré sous vide, le résidu est repris par de l'acétate d'éthyle, la phase organique est lavée à l'eau, séchée sur sulfate de magnésium, filtrée sur célite, et concentrée pour conduire au produit attendu.

### Stade 8 : 2-(Benzyloxy)-2[3-(3-méthoxy-3-oxopropyl)-2-oxo-1,2-dihydro-4-pyridinyl] butanoate de méthyle

257 ml d'une solution normale d'acide chlorhydrique sont ajoutés à une solution de 5 g (12.83 mmol) du composé décrit au stade précédent dans 80 ml de dioxane. Le milieu réactionnel est alors porté à reflux pendant 1 heure 30. Après retour à l'ambiante, le milieu est concentré sous vide, et le résidu est purifié par chromatographie sur gel de silice (éluant : dichlorométhane 97 / méthanol 3) pour conduire au produit attendu.

### Stade 9 : 5-(Benzyloxy)-5-éthyl-6-hydroxy-1-oxo-1,2,5,8-tétrahydro-7-isoquinolinecarboxylate de méthyle

A une suspension de 0,8 g (26,7 mmol) d'hydrure de sodium à 80 % dans 50 ml de THF agitée à 0°C sont ajoutés goutte à goutte 200 ml d'une solution de 4,7 g (12,1 mmol) du composé décrit au stade précédent dans le THF. Le milieu est ensuite agité 15 minutes à 0°C, 15 minutes à l'ambiante puis 1 heure au reflux avant d'être hydrolysé à 0°C par une solution normale d'acide chlorhydrique jusqu'à l'obtention d'un pH neutre. La phase aqueuse est extraite au dichlorométhane. La phase organique est lavée à l'eau, séchée sur sulfate de magnésium, et concentrée pour conduire au produit attendu.

### Stade 10 : 5-(Benzyloxy)-5-éthyl-7,8-dihydro-1,6(2H,5H)-isoquinolinedione

Une suspension de 16,1 g (45,3 mmol) du composé décrit au stade précédent dans 60 ml d'une solution normale de potasse est chauffée à reflux pendant 30 minutes. Le milieu réactionnel est neutralisé à 0°C par addition goutte à goutte d'une solution 2N d'acide chlorhydrique puis extrait au dichlorométhane. La phase organique est filtrée, séchée sur sulfate de magnésium et concentrée. Le produit attendu est obtenu après purification par chromatographie sur gel de silice (éluant : dichlorométhane/méthanol 100/0 à 96/4).

### Stade 11 : 5-Ethyl-5-hydroxy-7,8-dihydro-1,6(2H,5H)-isoquinolinedione

Une solution de 7,9 g (26,56 mmol) du composé décrit au stade précédent dans 800 ml de méthanol est agitée à pression atmosphérique et température ambiante sous atmosphère d'hydrogène en présence de 0,8 g de palladium sur charbon à 10 %. Après absorption de la quantité théorique d'hydrogène (environ 3 heures 30), le catalyseur est filtré, le filtrat est concentré, et le produit attendu est obtenu par cristallisation du résidu dans l'éther.

### PREPARATION H : 5-Ethyl-5-hydroxy-7-méthoxy-2,5,6,7-tétrahydro-1H-cyclopenta[c]pyridin-1-one

Une suspension de 2,2 g (11,95 mmol) du composé décrit au stade 2 de la préparation B dans 220 ml d'acide chlorhydrique IN est chauffée 1h30 à 80°C. Le milieu réactionnel est concentré, et le résidu est purifié par chromatographie sur silice (éluant : dichlorométhane/méthanol 95/5) pour conduire au composé attendu.

### PREPARATION I: (5S)-5-Ethyl-5-hydroxy-5,7-1H-cyclopenta[c]pyridine-1,6-(2H)-dione

### Stade 1 : 3-(3-Allyl-2-fluoro-4-pyridinyl)-3-pentanol

A une solution de 11,4 g (43,3 mmol) de 3-allyl-2-fluoro-4-iodopyridine (préparée selon le procédé décrit dans J.O.C., 1993, 58, pp.7832) dans 250 ml de THF, sont ajoutés à -75°C, 27 ml (43,3 mmol) de butyllithium 1,6 M dans l'hexane. Après 30 minutes d'agitation à -75°C, une solution 4,6 ml de 3-pentanone dans 100 ml de THF est ajoutée goutte à goutte. Le milieu réactionnel est agité pendant 6 heures à -75°C. Après retour à température ambiante, le milieu est hydrolysé par 100 ml d'eau et extrait à l'acétate d'éthyle. La phase organique est séchée, concentrée et purifiée par chromatographie sur silice (éluant : cyclohexane/acétate d'éthyle 8/2), pour conduire au produit attendu.

### Stade 2 : 3-Allyl-4-(1-éthyl-1-propényl)-2-fluoropyridine

A une solution de 1,4 g (6,3 mmol) du composé décrit au stade précédent, dans 50 ml de dichlorométhane, sont ajoutés 17,6 ml (126 mmol) de triéthylamine. La température est abaissée à 0°C et 2,3 ml (31,5 mmol) de chlorure de thionyle dans 20 ml de dichlorométhane sont ajoutés. Après 5 minutes d'agitation à température ambiante, le milieu réactionnel est versé sur 70 ml d'eau, puis extrait au dichlorométhane. la phase organique est séchée, concentrée, et purifiée par chromatographie sur silice (éluant : cyclohexane/acétate d'éthyle 9/1) pour conduire au produit attendu.

### Stade 3 : 5-Ethyl-1-fluoro-7H-cyclopenta[c]pyridine

Un mélange de 1 g (4,87 mmol) du composé décrit au stade précédent, et de 200 mg (0,24 mmol) de bis(tricyclohexylphosphine)benzylidène-dichlorure de ruthénium IV sont dissouts dans 20 ml de toluène. La solution est agitée à température ambiante pendant 15 heures puis filtrée. Le filtrat est concentré et purifié par chromatographie sur silice (éluant : cyclohexane/acétate d'éthyle 8/2) pour conduire au produit attendu.

### Stade 4 : (5S)-5-Ethyl-1-fluoro-6,7-dihydro-5H-cyclopenta[c]pyridine-5,6-diol

A une solution de 80 mg (0,01 mmol) de (DHQD)2-Pyr dans 50 ml d'alcool tert-butylique, sont ajoutés successivement 50 ml d'eau distillée, 9 g (27,3 mmol) de ferricyanure de potassium, 3,6 g (28 mmol) de carbonate de potassium, 10 mg (0,03 mmol) de dihydrate d'osmate de potassium et 0,85 g (8,9 mmol) de méthanesulfonamide. Après 3 minutes d'agitation à température ambiante, le mélange est refroidi à 0°C et une solution de 1,4 g (8,6 mmol) du composé décrit au stade précédent dans 5 ml d'alcool tertbutylique est ajoutée. Le mélange réactionnel est agité 2 jours à 0°C. Le milieu est extrait à l'acétate d'éthyle, les phases organiques sont séchées, concentrées et purifiées par chromatographie sur silice (éluant : cyclohexane/acétate d'éthyle 5/5) pour conduire au produit attendu.

### Stade 5 : (5S)-5-Ethyl-1-fluoro-5-hydroxy-5,7-dihydro-6H-cyclopenta[c]pyridine-6-one

A un mélange de 1,1 g (5,5 mmol) du composé décrit au stade précédent et de 0,48 ml (5,5 mmol) de chlorure d'oxalyle dans 100 ml de dichlorométhane à -78°C est ajouté 0,82 ml de diméthylsulfoxyde. Après 20 minutes à -78°C, 4 ml (28,4 mmol) de triéthylamine sont ajoutés et le mélange est agité 15 minutes à basse température, et ramené à l'ambiante. La réaction est hydrolysée par 100 ml d'eau, et extraite au dichlorométhane. La phase organique est lavée par une solution aqueuse d'acide chlorhydrique à 1 %, puis par une solution aqueuse saturée de dicarbonate de sodium. Après séchage et concentration, le produit attendu est obtenu.

### Stade 6 : (5S)-5-Ethyl-5-hydroxy-5,7-1H-cyclopenta[c]pyridine-1,6-(2H)-dione

Le produit attendu est obtenu selon le procédé décrit au stade 4 de la préparation F en utilisant comme produit de départ le composé décrit au stade précédent.

### PREPARATION J: 5-Ethyl-5-hydroxy-5,8-dihydro-1-(2H)-isoquinolinone

### Stade 1 : 1-(3-Allyl-2-fluoro-4-pyridinyl)-1-propanol

Le produit attendu est obtenu selon le procédé décrit au stade 1 de la préparation I en remplaçant la 3-pentanone par le propanal.

### Stade 2 : 1-(3-Allyl-2-fluoro-4-pyridinyl)-1-propanone

A une solution de 0,5 g (2,56 mmol) du composé décrit au stade précédent dans 25 ml de dichlorométhane, à 0°C, sont ajoutés 0,5 g de célite, 0,2 g de tamis moléculaire 4Å, et 0,58 g (2,69 mmol) de chlorochromate de pyridinium. Après retour à température ambiante, le mélange est agité 15 heures, puis filtré. Le filtrat est concentré et purifié par chromatographie sur silice (éluant : dichlorométhane/acétone, 97/3) pour conduire au produit attendu.

### Stade 3 : 3-(3-Allyl-2-fluoro-4-pyridinyl)-1-pentèn-3-ol

A une solution de 0,32 g (1,66 mmol) du composé décrit au stade précédent dans 10 ml de THF, sont ajoutés à 0°C, 2,1 ml d'une solution 1M de bromure de vinylmagnésium dans le THF. Après 45 minutes d'agitation à 0°C, le milieu est hydrolysé par une solution d'acide chlorhydrique 1M, et extrait à l'éther. Les phases organiques sont lavées par une solution aqueuse saturée en hydrogénocarbonate de sodium, séchées sur sulfate de magnésium et concentrées. Le résidu est purifié par chromatographie sur gel de silice (éluant dichlorométhane/acétone, 95/5) pour conduire au produit attendu.

### Stade 4 : 5-Ethyl-1-fluoro-5,8-dihydro-5-isoquinolinol

Le produit attendu est obtenu selon le procédé décrit au stade 3 de la préparation I en utilisant comme produit de départ le composé décrit au stade précédent.

### Stade 5 : 5-Ethyl-5-hydroxy-5,8-dihydro-1-(2H)-isoquinolinone

A une solution de 0,5 g (2,59 mmol) du produit décrit au stade précédent dans 25 ml de tertbutanol sont ajoutés 5 g (45 mmol) de tertiobutylate de potassium. Après 16 heures d'agitation à reflux, le milieu réactionnel refroidit est hydrolysé par 50 ml d'eau et acidifié par une solution d'acide chlorhydrique 2M. Le précipité obtenu est filtré et séché pour conduire au produit attendu.

### PREPARATION K : 5-Ethyl-5-hydroxy-5,8-dihydro-1,6,7-(2H)-isoquinolinetrione

### Stade 1 : 5-Ethyl-5,6,7-trihydroxy-5,6,7,8-tetrahydro-1-(2H)-isoquinolinone

A une solution de 2 g (10,4 mmoles) du composé décrit dans la préparation J dans un mélange de 70 ml de dioxane et 30 ml d'eau sont ajoutés à température ambiante 4 ml d'une solution 2,5% de tétroxyde d'osmium dans le terbutanol, puis 1,5 g du N-oxyde de la N-méthyl-morpholine (12,8 mmoles). Après une nuit d'agitation à température ambiante, le milieu est extrait à l'acétate d'éthyle, les phases organiques sont séchées et concentrées pour conduire au produit attendu.

### Stade 2 : 5-Ethyl-5-hydroxy-5,8-dihydro-1,6,7-(2H)-isoquinolinetrione

A un mélange de 1 g (4,44 mmoles) du composé décrit au stade précédent et de 0,78 ml (8,88 mmoles) de chlorure d'oxalyle dans 100 ml de dioxane à -78°C est ajouté 1,30 ml de diméthylsulfoxyde (18,2 mmoles). Après 30 minutes à -78°C, 6 ml (42,6 mmoles) de triéthylamine sont ajoutés. Après 15 minutes à -78°C, le mélange est ramené à température ambiante. La réaction est hydrolysée par 100 ml d'eau, et extraite à l'acétate d'éthyle. La phase organique est lavée par une solution 1 N d'acide chlorhydrique puis par une solution aqueuse saturée de bicarbonate de sodium. Après séchage et concentration, le produit attendu est obtenu.

### PREPARATION L : 7-Ethyl-7-hydroxy-2,4,7,7a-tétrahydroxireno[2,3-g]isoquinolin-3(1aH)-one

A une solution de 1 g (5,2 mmoles) du produit décrit dans la préparation J dans 100 ml de dioxane est ajouté à 0°C 1,17 g (6,8 mmoles) d'acide m-chloroperbenzoïque. Après agitation 1 heure à température ambiante, la solution est diluée avec 100 ml d'acétate d'éthyle et lavée avec une solution aqueuse saturée en hydrogénocarbonate de sodium. Après séchage sur sulfate de magnésium et évaporation des solvants, on obtient le produit attendu.

### PREPARATION M : 5-Ethyl-5-hydroxy-2,5,6,9-tétrahydro-1H-cyclohepta[c]pyridin-1-one

Le produit attendu est obtenu selon le procédé décrit dans la préparation J en remplaçant dans le stade 3 le bromure de vinyl magnésium par le bromure d'allyl magnésium.

### PREPARATION N : 5-Ethyl-5-hydroxy-5,6,8,9-tétrahydro-1H-cyclohepta[c]pyridine -1,7(2H)-dione

### Stade 1 : (2-Fluoro-4-iodo-3-pyridinyl)méthanol

A une solution de 4 g (16 mmol) de 2-fluoro-4-iodo-3-pyridinecarbaldéhyde (décrit dans J.O.C., 1993, 58, pp.7832) dans 100 ml de tétrahydrofurane, sont ajoutés 0,72 g (19,2 mmol) de borohydrure de sodium. Le mélange est agité à température ambiante pendant 4 heures, puis hydrolysé. Le milieu réactionnel est extrait à l'acétate d'éthyle. Les phases organiques sont séchées, et concentrées pour conduire au produit attendu.

### Stade 2 : 2-Fluoro-4-iodo-3-[(2-méthoxyéthoxy)méthyl]pyridine

A une solution de 3,5 g (14 mmol) du composé décrit au stade précédent dans 20 ml de dichlorométhane, sont ajoutées successivement une solution de 2,1 g (16,8 mmol) de chlorométhoxy-2-méthoxyéthane dans 10 ml de dichlorométhane, puis une solution de 2,2 g (16,8 mmol) d'éthyldiisopropylamine et de 180 mg (1,4 mmol) de 4-diméthylaminopyridine dans 10 ml de dichlorométhane. Le mélange est maintenu sous agitation pendant 18 heures, puis hydrolysé. Le milieu est extrait au dichlorométhane, et la phase organique est lavée jusqu'à neutralité par une solution aqueuse saturée en chlorure de sodium, séchée et concentrée pour conduire au produit attendu.

### Stade 3 : 1-{2-Fluoro-3-[(2-méthoxyéthoxy)méthyl]-4-pyridinyl}-1-propanol

Le produit attendu est obtenu selon le procédé décrit au stade 2 de la préparation G, en utilisant comme produit de départ le composé décrit au stade précédent et comme agent électrophile le propionaldéhyde.

### Stade 4 : 1-{2-Fluoro-3-[(2-méthoxyéthoxy)méthyl]-4-pyridinyl}-1-propanone

Le produit attendu est obtenu selon le procédé décrit au stade 1 de la préparation E en utilisant comme produit de départ le composé décrit au stade précédent.

### Stade 5 : 3-{2-Fluoro-3-[(2-méthoxyéthoxy)méthyl]-4-pyridinyl}-3-hydroxypentanoate de méthyle

A une suspension de 2,16 g (33 mmol) de zinc, préalablement activé par un traitement à l'acide chlorhydrique 6M, dans 5 ml de THF à reflux, on ajoute goutte à goutte une solution de 3 g (10,2 mmol) du composé décrit au stade précédent et de 5,1 g (33 mmol) de bromoacétate de méthyle dans 40 ml de THF, de manière à entretenir le reflux. L'addition terminée, le milieu est agité à reflux pendant 1 heure, puis refroidi et hydrolysé à l'aide d'une solution aqueuse saturée en chlorure d'ammonium. Après extraction à l'éther, la phase organique est séchée, concentrée et purifiée par chromatographie sur silice (éluant : dichlorométhane/acétone, 95/5) pour conduire au produit attendu.

### Stade 6 : 3-[2-Fluoro-3-(hydroxyméthyl)-4-pyridinyl]-3-hydroxypentanoate de méthyle

Une solution de 3,5 g (10,1 mmol) du composé préparé au stade précédent dans 20 ml de THF et 20 ml d'HCl 2N est agitée 3 heures à température ambiante. Le milieu réactionnel est décanté, la phase aqueuse est extraite à l'acétate d'éthyle, les phases organiques réunies sont lavées jusqu'à neutralité à l'aide d'une solution aqueuse saturée de chlorure de sodium, séchées sur sulfate de magnésium et le produit attendu est obtenu par évaporation des solvants.

### Stade 7 : 3-(2-Fluoro-3-formyl-4-pyridinyl)-3-hydroxypentanoate de méthyle

A une solution de 3 g (11,6 mmol) du composé décrit au stade précédent dans 50 ml de dichlorométhane, sont ajoutés 1,2 g (13,9 mmol) de dioxyde de manganèse. Après 2 heures d'agitation à température ambiante, le milieu réactionnel est filtré sur célite, et le filtrat concentré pour conduire au produit attendu.

### Stade 8 : 3-[4-(1-Ethyl-1-hydroxy-3-méthoxy-3-oxopropyl)-2-fluoro-3-pyridinyl]-2-acrylate de méthyle

Le produit attendu est obtenu selon le procédé décrit au stade 6 de la préparation G en utilisant comme produit de départ le composé décrit au stade précédent.

### Stade 9 : 3-[2-Fluoro-3-(3-méthoxy-3-oxopropyl)-4-pyridinyl]-3-hydroxypentanoate de méthyle

Le produit attendu est obtenu selon le procédé décrit au stade 7 de la préparation G en utilisant comme produit de départ le composé décrit au stade précédent.

### Stade 10 : 3-[2-Fluoro-3-(3-méthoxy-3-oxopropyl)-2-oxo-1,2-dihydro-4-pyridinyl]-3-triméthylsilyloxy-pentanoate de méthyle

Une solution de 2,7 ml (15 mmol) de triflate de triméthylsilyle dans 5 ml de chlorure de méthylène est ajoutée goutte à goutte à 0°C à une solution de 3,1 g (10 mmol) du composé préparé au stade précédent et de 3 g (30 mmol) de triéthylamine dans 20 ml de chlorure de méthylène. Le milieu réactionnel est agité 2 heures à 0°C, puis hydrolysé à l'aide de 20 ml d'eau. La phase organique est décantée, séchée sur sulfate de magnésium. Le produit attendu est obtenu par évaporation du solvant.

### Stade 11 : 5-Ethyl-1-fluoro-7-oxo-5-triméthylsilanyloxy-6,7,8,9-tétrahydro-5H-cyclohepto[c]pyridine-8-carboxylate de méthyle

Le produit attendu est obtenu selon le procédé décrit au stade 9 de la préparation G en utilisant comme produit de départ le composé décrit au stade précédent.

### Stade 12 : 5-Ethyl-5-hydroxy-5,6,8,9-tétrahydro-1H-cyclohepta[c]pyridine-1,7(2H)-dione

Une suspension de 1,7 g (5 mmol) des composés préparés au stade précédent dans 20 ml d'une solution normale de potasse est chauffée à reflux 2 heures. Le milieu réactionnel refroidi à l'ambiante est acidifié par ajout de 40 ml d'une solution 6N d'acide chlorhydrique, agité 30 minutes à l'ambiante puis à 80°C 1 heure. Après refroidissement le milieu est extrait au chlorure de méthylène. La phase organique séchée sur sulfate de magnésium et concentrée fournit le composé attendu.

### PREPARATION O: 5-Ethyl-5-hydroxy-2,5,6,8-tétrahydro-1,7-isoquinolinedione

A une solution de 1 g (4,8 mmoles) du produit décrit dans la préparation L dans 100 ml de dioxane est ajouté à -78°C 2 ml du complexe trifluorure de bore-éther diéthylique (16,7 mmol). Après retour à température ambiante, le mélange est agité 20 heures, puis versé sur un mélange acétate d'éthyl/eau. Les phases organiques sont séchées sur sulfate de magnésium. L'évaporation des solvants fournit le produit attendu.

### EXEMPLE 1 : 9-Hydroxy-9,12-dihydro-7H-cyclopenta[6,7]indolizino[1,2-b] quinoline-7,10(8H)-dione

### Stade a : 2-[(2-Bromo-3-quinolinyl)méthyl]-7-hydroxy-6,7-dihydro-1H-cyclopenta[c] pyridine-1,5(2H)-dione

A une solution agitée à 0°C de 0,6 mmol (100 mg) du composé décrit dans la préparation A dans 20 ml d'un mélange de DME/DMF (16/4 (v/v)), sont ajoutées par petites fractions 0,6 mmol (18 mg) de NaH en dispersion à 80 % dans l'huile minérale. Le milieu est agité à 0°C pendant 10 minutes avant d'ajouter à cette température 2,4 mmol (210 mg) de LiBr. Après retour à température ambiante, le milieu réactionnel est agité pendant ¼ d'heure avant d'ajouter 0,66 mmol (200 mg) de 2-bromo-3-bromométhylquinoléine. Le mélange réactionnel est ensuite chauffé à 80°C pendant 20 heures puis après retour à l'ambiante versé sur un large excès d'eau. Le précipité formé est filtré puis lavé à l'éther, pour conduire au produit attendu.

### Stade b : 9-Hydroxy-9,12-dihydro-7H-cyclopenta[6,7]indolizino[1,2-b]quinoline-7,10(8H)-dione

Sous atmosphère inerte, sont mélangées successivement 0,98 mmol (380 mg) du composé décrit au stade précédent, 3,2 mmol (319 mg) d'acétate de potassium, 1,57 mmol (505 mg) de bromure de tétrabutylammonium et 17 mmol (38 mg) d'acétate de palladium dans 45 mL d'acétonitrile. Le mélange est ensuite porté à reflux pendant 4 heures puis filtré à chaud. Le résidu est lavé avec une solution chaude de dichlorométhane et méthanol. Le filtrat est ensuite concentré, purifié par chromatographie sur silice (éluant : dichlorométhane/méthanol 98/2) pour conduire au produit attendu.
*Spectre de masse :* LC-MS : m/z : 305 (MH⁺) ; 287 (MH⁺-H₂O)

### EXEMPLE 2 : 7-Ethyl-7,8-dihydroxy-7,8,9,12-tétrahydro-10H-cyclopenta[6,7] indolizino[1,2-b]quinolin-10-one

Le produit attendu est obtenu selon le procédé décrit dans l'exemple 1, en utilisant comme produit de départ le composé décrit dans la préparation B.

### EXEMPLE 3: 7-Ethyl-7,8-dihydroxy-7,12-dihydro-10H-cyclopenta[6,7]indolizino [1,2-b]quinolin-10-one

Le produit attendu est obtenu selon le procédé décrit dans l'exemple 1, en utilisant comme produit de départ le composé décrit dans la préparation C.

### EXEMPLE 4 : 7-Ethyl-7-hydroxy-7H-cyclopenta[6,7]indolizino[1,2-b]quinoline-8,9,10(12H)-trione

Le produit attendu est obtenu selon le procédé décrit dans l'exemple 1, en utilisant comme produit de départ le composé décrit dans la préparation D.

### EXEMPLE 5: 7-Ethyl-7-hydroxy-7H-cyclopenta[6,7]indolizino[1,2-b]quinoline-9,10(8H,12H)-dione

Le produit attendu est obtenu selon le procédé décrit dans l'exemple 1, en utilisant comme produit de départ le composé décrit dans la préparation E.

### EXEMPLE 6a : 7-Ethyl-7-hydroxy-8-[2-(1,3dioxolan)yl]-9,12-dihydro-7H-cyclopenta[6,7]indolizino[1,2-b]quinoline-10-one

Le produit attendu est obtenu selon le procédé décrit dans l'exemple 1, en utilisant comme produit de départ le composé décrit dans la préparation F. *Spectre de masse :* (MH⁺) m/z = 377

### EXEMPLE 6b : 7-Ethyl-7-hydroxy-9,12-dihydro-7H-cyclopenta[6,7]indolizino [1,2-b]quinoline-8,10-dione

Une solution de 1 g (2,66 mmol) du composé préparé dans l'exemple 6a dans 10 ml d'acide trifluoroacétique et 1 ml d'eau est chauffée 2 heures à 80°C. Le milieu est concentré sous vide, le résidu est plongé dans 20 ml d'eau, le précipité est filtré, séché, et repris dans 10 ml d'alcool chaud. Le produit attendu est obtenu par filtration et séchage de l'insoluble. *Spectre de masse :* (MH⁺) m/z = 333

Les composés des exemples 9,10,11,12,13,14,15 ont été obtenus de la même manière.

### EXEMPLE 7 : 7-Ethyl-7-hydroxy-10,13-dihydrobenzo[6,7]indolizino[1,2-b] quinoline-8,11(7H,9H)-dione

Le produit attendu est obtenu selon le procédé décrit dans l'exemple 1, en utilisant comme produit de départ le composé décrit dans la préparation G.
*Spectre de masse :* (MH⁺) m/z = 347

### EXEMPLE 8: 7-Ethyl-7-hydroxy-9-méthoxy-7,8,9,12-tétrahydro-10H-cyclopenta [6,7]indolizino[1,2-b]quinoline-10-one

Le produit attendu est obtenu selon le procédé décrit dans l'exemple 1, en utilisant comme produit de départ le composé décrit dans la préparation H.
*Spectre de masse :* (MH⁺) m/z = 349

### EXEMPLE 9: 3-Chloro-7-éthyl-7-hydroxy-2-méthyl-9,12-dihydro-7H-cyclopenta [6,7]indolizino[1,2-b]quinoline-8,10-one

Le produit attendu est obtenu selon le procédé décrit dans l'exemple 6 (6a + 6b), en utilisant comme produit de départ le composé décrit dans la préparation F, et en remplaçant la 2-bromo-3-bromométhylquinoléine par la 2-bromo-3-bromométhyl-7-chloro-6-méthylquinoléine.
*Spectre de masse :* (MH⁺) m/z = 381

### EXEMPLE 10 : 2,3-Diméthyl-7-éthyl-7-hydroxy-9,12-dihydro-7H-cyclopenta[6,7] indolizino[1,2-b]quinoline-8,10-dione

Le produit attendu est obtenu selon le procédé décrit dans l'exemple 6 (6a + 6b), en utilisant comme produit de départ le composé décrit dans la préparation F, et en remplaçant la 2-bromo-3-bromométhylquinoléine par la 2-bromo-3-bromométhyl-6,7-diméthylquinoléine.
*Spectre de masse :* (MH⁺) m/z = 361

### EXEMPLE 11 : 7-Ethyl-7-hydroxy-2,3-méthylènedioxy-9,12-dihydro-7H-cyclopenta [6,7]indolizino[1,2-b]quinoline-8,10-dione

Le produit attendu est obtenu selon le procédé décrit dans l'exemple 6 (6a + 6b), en utilisant comme produit de départ le composé décrit dans la préparation F, et en remplaçant la 2-bromo-3-bromométhylquinoléine par la 2-bromo-3-bromométhyl-6,7-méthylènedioxyquinoléine.
*Spectre de masse :* (MH⁺) m/z = 377

### EXEMPLE 12 : 2,3-Difluoro-7-éthyl-7-hydroxy-9,12-dihydro-7H-cyclopenta[6,7] indolizino[1,2-b]quinoline-8,10-dione

Le produit attendu est obtenu selon le procédé décrit dans l'exemple 6 (6a + 6b), en utilisant comme produit de départ le composé décrit dans la préparation F, et en remplaçant la 2-bromo-3-bromométhylquinoléine par la 2-bromo-3-bromométhyl-6,7-difluoroquinoléine.
*Spectre de masse :* (MH⁺) m/z = 369

### EXEMPLE 13 : 2-Chloro-7-éthyl-7-hydroxy-9,12-dihydro-7H-cyclopenta[6,7] indolizino[1,2-b]quinoline-8,10-dione

Le produit attendu est obtenu selon le procédé décrit dans l'exemple 6 (6a + 6b), en utilisant comme produit de départ le composé décrit dans la préparation F, et en remplaçant la 2-bromo-3-bromométhylquinoléine par la 2-bromo-3-bromométhyl-6-chloroquinoléine.
*Spectre de masse :* (MH⁺) m/z = 367

### EXEMPLE 14 : 7-Ethyl-7-hydroxy-2-méthoxy-9,12-dihydro-7H-cyclopenta[6,7] indolizino[1,2-b]quinoline-8,10-dione

Le produit attendu est obtenu selon le procédé décrit dans l'exemple 6 (6a + 6b), en utilisant comme produit de départ le composé décrit dans la préparation F, et en remplaçant la 2-bromo-3-bromométhylquinoléine par la 2-bromo-3-bromométhyl-6-méthoxyquinoléine.
*Spectre de masse :* (MH⁺) m/z = 363

### EXEMPLE 15 : 2-Chloro-7-éthyl-7-hydroxy-3-méthyl-9,12-dihydro-7H-cyclopenta [6,7]indolizino[1,2-b]quinoline-8,10-dione

Le produit attendu est obtenu selon le procédé décrit dans l'exemple 6 (6a + 6b), en utilisant comme produit de départ le composé décrit dans la préparation F, et en remplaçant la 2-bromo-3-bromométhylquinoléine par la 2-bromo-3-bromométhyl-6-chloro-7-méthylquinoléine.

### EXEMPLE 16 : (7S)-7-Ethyl-7-hydroxy-9,12-dihydro-7H-cyclopenta[6,7]indolizino [1,2-b]quinoline-8,10-dione

Le produit attendu est obtenu selon le procédé décrit dans l'exemple 1, en utilisant comme produit de départ le composé décrit dans la préparation I.

### EXEMPLE 17: 7-Ethyl-7-hydroxy-10,13-dihydrobenzo[6,7]indolizino[1,2-b] quinolin-11(7H)-one

Le produit attendu est obtenu selon le procédé décrit dans l'exemple 1, en utilisant comme produit de départ le composé décrit dans la préparation J.

### EXEMPLE 18 : 7-Ethyl-7-hydroxy-10,13-dihydrobenzo[6,7]indolizino[1,2-b] quinoline-8,9,11(7H)-trione

Le produit attendu est obtenu selon le procédé décrit dans l'exemple 1, en utilisant comme produit de départ le composé décrit dans la préparation K.

### EXEMPLE 19 : 2-Ethyl-2-hydroxy-1a,10,13,13a-tétrahydro[1]benzoxirèno[3',4':6,7] indolizino[1,2-b]quinolin-12(2H)-one

Le produit attendu est obtenu selon le procédé décrit dans l'exemple 1, en utilisant comme produit de départ le composé décrit dans la préparation L.

### EXEMPLE 20 : 7-Ethyl-7-hydroxy-7,8,10,13-tétrahydrobenzo[6,7]indolizino[1,2-b] quinoline-9,11-dione

Le produit attendu est obtenu selon le procédé décrit dans l'exemple 1, en utilisant comme produit de départ le composé décrit dans la préparation O.

### EXEMPLE 21 : 7-Ethyl-7-hydroxy-7,8,11,14-tétrahydro-12H-cyclohepta[6,7] indolizino[1,2-b]quinolin-12-one

Le produit attendu est obtenu selon le procédé décrit dans l'exemple 1, en utilisant comme produit de départ le composé décrit dans la préparation M.

### EXEMPLE 22 : 7-Ethyl-7-hydroxy-11,14-dihydro-7H-cyclohepta[6,7]indolizino [1,2-b]quinoline-9,12(8H,10H)-dione

Le produit attendu est obtenu selon le procédé décrit dans l'exemple 1, en utilisant comme produit de départ le composé décrit dans la préparation N.

### EXEMPLE 23 : 2,3-Difluoro-7-éthyl-7-hydroxy-10,13-dihydrobenzo [6,7]indolizino [1,2-b]quinoline-8,11(7H,9H)-dione

Le produit attendu est obtenu selon le procédé décrit dans l'exemple 1, en utilisant comme produit de départ le composé décrit dans la préparation G, et en remplaçant la 2-bromo-3-bromoéthylquinoléine par la 2-bromo-3-bromométhyl-6,7-difluoroquinoléine.
*Spectre de masse :* (MH⁺) m/z = 383

### ETUDE PHARMACOLOGIQUE

### EXEMPLE A : Activité in vitro

La leucémie marine L1210 a été utilisée in vitro. Les cellules sont cultivées dans du milieu de culture RPMI 1640 complet contenant 10 % de sérum de veau foetal, 2 mM de glutamine, 50 U/ml de péniciline, 50 µg/ml de streptomycine et 10 mM d'Hepes, pH : 7,4. Les cellules sont réparties dans des microplaques et exposées aux composés cytototoxiques pendant 4 temps de doublement, soit 48 heures (L1210). Le nombre de cellules viables est ensuite quantifié par un essai colorimétrique, le Microculture Tetrazolium Assay (J. Cannichael et al., Cancer Res. ; 47, 936-942, (1987)). Les résultats sont exprimés en IC₅₀, concentration en cytotoxique qui inhibe à 50 % la prolifération des cellules traitées.

Il apparaît que les composés de l'invention sont de puissants cytotoxiques, les IC₅₀ étant nettement inférieurs à 10⁻⁶ M.

### EXEMPLE B : Composition pharmaceutique

Formule de préparation pour 1000 comprimés dosés à 10 mg :

| | |
|---|---|
| Composé de l'exemple 5 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 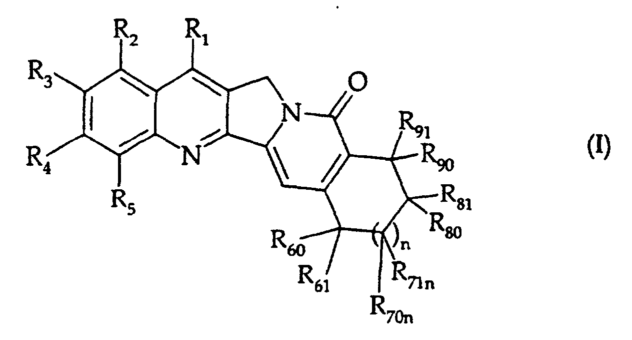 dans laquelle :
• n vaut 0, 1 ou 2,
• R₁, R₂, R₃, R₄ et R₅ sont choisis indépendamment parmi un atome d'hydrogène, d'halogène, un groupement alkyle, alkényle, alkynyle, perhalogénoalkyle, cycloalkyle(C₃-C₁₁), (C₃-C₁₁) cycloalkylalkyle, hydroxy, hydroxyalkyle, alkoxy, alkoxyalkyle, alkoxycarbonyle, acyloxy, carboxy, nitro, cyano, aminocarbonyle (éventuellement substitué sur l'atome d'azote par un ou deux groupements alkyle), et les groupements (CH₂)ₚ-NRₐR_{b}, et -O-C(O)-N-RₐR_{b}, dans lesquels p est un entier compris entre 0 et 6, et Rₐ et R_{b} représentent indépendamment un atome d'hydrogène, un groupement alkyle, cycloalkyle (C₃-C₁₁), (C₃-C₁₁) cycloalkylalkyle, acyle, aryle éventuellement substitué, arylalkyle éventuellement substitué, ou bien Ra et Rb forment ensemble avec l'atome d'azote qui les porte un groupement pyrrolyle, pipéridinyle, ou pipérazinyle, chacun de ces groupements cycliques pouvant être éventuellement substitué,
ou bien deux groupements R₂, R₃, R₄ et R₅ adjacents forment ensemble avec les atomes de carbone qui les portent un groupement -O-(CH₂)ₜ-O, t étant un entier compris entre 1 et 3 inclus,
• R₆₀, R₇₀ₙ, R₈₀ et R₉₀ représentent indépendamment un atome d'hydrogène, un groupement hydroxy, alkoxy, ou un groupement O-(CO)-X ou O-(CO)-NXW dans lesquels X et W représentent indépendamment un groupement alkyle, alkényle, alkynyle, cycloalkyle (C₃-C₁₁), (C₃-C₁₁) cycloalkylalkyle, aryle éventuellement substitué, ou arylalkyle éventuellement substitué,
• R₆₁, R₇₁ₙ, R₈₁ et R₉₁ représentent indépendamment un atome d'hydrogène, un groupement alkyle, alkényle, alkynyle, ou pris deux à deux sur des carbones adjacents forment ensemble une liaison, ou un groupement oxirane, ou bien deux groupements géminaux (R₆₀ et R₆₁) et/ou (R₇₀ₙ et R₇₁ₙ) et/ou (R₈₀ et R₈₁) et/ou (R₉₀ et R₉₁) forment ensemble un groupement oxo ou un groupement -O-(CH₂)ₜ₁-O, t₁ étant un entier compris entre 1 et 3 inclus,
à la condition que R₆₀, R₆₁, R₇₀ₙ, R₇₁ₙ, R₈₀, R₈₁, R₉₀, R₉₁ ne représentent pas tous un atome d'hydrogène,
leurs énantiomères, diastéréoisomères, ainsi que leurs sels d'addition à un acide ou à une base pharmaceutiquement acceptable,
étant entendu que :
- le terme alkyle désigne une chaîne de 1 à 6 atomes de carbone, linéaire ou ramifié,
- le terme alkényle désigne une chaîne de 2 à 6 atomes de carbone, linéaire ou ramifié et contenant de 1 à 3 doubles liaisons,
- le terme alkynyle désigne une chaîne de 2 à 6 atomes de carbone, linaire ou ramifié et contenant de 1 à 3 triples liaisons,
- le terme alkoxy désigne un radical alkyle-oxy linéaire ou ramifié contenant de 1 à 6 atomes de carbone,
- le terme acyle désigne un radical alkyle-carbonyle linéaire ou ramifié contenant de 1 à 6 atomes de carbone,
- le terme aryle représente un groupement phényle ou naphtyle,
- l'expression "substitué" lorsqu'elle concerne les groupements aryle ou arylalkyle signifie que les groupements concernés sont substitués par un ou plusieurs atomes d'halogène, et/ou groupement alkyles, alkoxy, hydroxy, cyano, nitro, et/ou amino (éventuellement substitué par un ou deux groupements alkyle),
- l'expression "substitué" lorsqu'elle concerne les groupements pyrrolyle, pipéridinyle, ou pipérazinyle signifie que les groupements concernés sont substitués par un ou plusieurs groupements alkyle, alkoxy, aryle, arylalkyle, aryloxy, et/ou aryloxyalkyle.

2. Composés de formule (I) selon la revendication 1 pour lesquels n vaut 0, leurs énantiomères, diastéréoisomères ainsi que leurs sels d'addition à un acide ou à une base pharmaceutiquement acceptable.

3. Composés de formule (I) selon la revendication 1 pour lesquels n vaut 1, leurs énantiomères, diastéréoisomères ainsi que leurs sels d'addition à un acide ou à une base pharmaceutiquement acceptable.

4. Composés de formule (I) selon la revendication 1 pour lesquels n vaut 2, leurs énantiomères, diastéréoisomères ainsi que leurs sels d'addition à un acide ou à une base pharmaceutiquement acceptable.

5. Composés de formule (I) selon la revendication 1 pour lesquels R₆₀ représente un groupement hydroxy et R₆₁ représente un groupement alkyle, leurs énantiomères, diastéréoisomères ainsi que leurs sels d'addition à un acide ou à une base pharmaceutiquement acceptable.

6. Composés de formule (I) selon la revendication I pour lesquels R₈₀ et R₈₁ forment ensemble un groupement oxo, leurs énantiomères, diastéréoisomères ainsi que leurs sels d'addition à un acide ou à une base pharmaceutiquement acceptable.

7. Composés de formule (I) selon la revendication I pour lesquels R₉₀ et R₉₁ forment ensemble un groupement oxo, leurs énantiomères, diastéréoisomères ainsi que leurs sels d'addition à un acide ou à une base pharmaceutiquement acceptable.

8. Composés de formule (I) selon la revendication 1 pour lesquels R₈₀ et R₈₁ ainsi que R₉₀ et R₉₁ forment deux groupements oxo, leurs énantiomères, diastéréoisomères ainsi que leurs sels d'addition à un acide ou à une base pharmaceutiquement acceptable.

9. Composés de formule (I) selon la revendication I pour lesquels R₁ représente un atome d'hydrogène, leurs énantiomères, diastéréoisomères ainsi que leurs sels d'addition à un acide ou à une base pharmaceutiquement acceptable.

10. Composés de formule (I) selon la revendication 1 pour lesquels R₂, R₃, R₄ et R₅ sont choisis parmi un atome d'hydrogène, d'halogène, un groupement alkyle, alkoxy, ou bien deux de ces groupements lorsqu'ils sont reliés à deux carbones adjacents forment ensemble un groupement méthylènedioxy ou éthylènedioxy, leurs énantiomères, diastéréoisomères ainsi que leurs sels d'addition à un acide ou à une base pharmaceutiquement acceptable.

11. Composés de formule (I) selon la revendication 1 pour lesquels R₁, R₂ et R₅ représentent chacun un atome d'hydrogène, R₃ et R₄ sont choisis parmi un atome d'hydrogène, d'halogène, un groupement alkyle, alkoxy, ou bien forment ensemble un groupement méthylènedioxy, R₆₀, R₇₀ₙ, R₈₀ et R₉₀ représentent indépendamment un atome d'hydrogène, un groupement hydroxy, ou alkoxy, et R₆₁, R₇₁ₙ, R₈₁ et R₉₁ représentent indépendamment un atome d'hydrogène, un groupement alkyle, ou pris deux à deux sur des carbones adjacents forment ensemble une liaison ou un groupement oxirane, ou bien deux groupements géminaux (R₆₀ et R₆₁) et/ou (R₇₀ₙ et R₇₁ₙ) et/ou (R₈₀ et R₈₁) et/ou (R₉₀ et R₉₁) forment ensemble un groupement oxo, leurs énantiomères, diastéréoisomères ainsi que leurs sels d'addition à un acide ou à une base pharmaceutiquement acceptable.

12. Composé de formule (I) selon la revendication 1 qui est la :
- 3-Chloro-7-éthyl-7-hydroxy-2-méthyl-9,12-dihydro-7*H*-cyclopenta[6,7] indolizino[1,2-b]quinoline-8,10-one

13. Composé de formule (I) selon la revendication 1 qui est la
- 2,3-Difluoro-7-éthyl-7-hydroxy-9,12-dihydro-7*H*-cyclopenta[6,7]indolizino [1,2-b]quinoline-8,10-dione

14. Procédé de préparation des composés de formule (I) selon la revendication 1 **caractérisé en en ce que** l'on utilise comme produit de départ un composé de formule (II) : 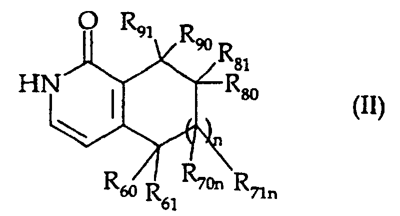 dans laquelle n, R₆₀, R₆₁, R₇₀ₙ, R₇₁ₙ, R₈₀, R₈₁, R₉₀, R₉₁ sont tels que définis dans la formule (I),
qui est condensé, en milieu basique, sur un composé de formule (III) : 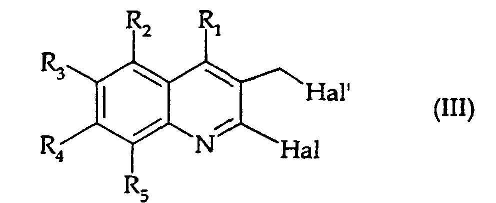 dans laquelle R₁, R₂, R₃, R₄, et R₅ sont tels que définis dans la formule (I), et Hal et Hal' représentent indépendamment un atome d'halogène,
ou bien, sur un composé de formule (III') : 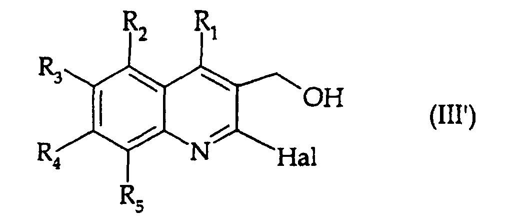 dans laquelle R₁, R₂, R₃, R₄, R₅ et Hal sont tels que définis précédemment,
pour conduire à un composé de formule (IV) : 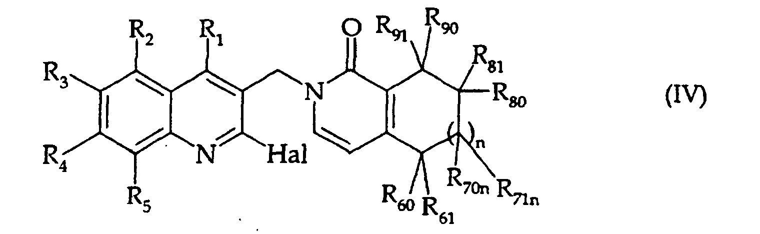 dans laquelle R₁, R₂, R₃, R₄, R₅, n, R₆₀, R₆₁, R₇₀ₙ, R₇₁ₙ, R₈₀, R₈₁, R₉₀, R₉₁ et Hal sont tels que définis précédemment,
composé (IV) qui subit une réaction de cyclisation intramoléculaire catalysé par un dérivé du palladium, pour conduire au composé de formule (I),
étant entendu, dans le but de simplifier le procédé ci-dessus, que les groupements réactifs présents en R₆₀, R₆₁, R₇₀ₙ, R₇₁ₙ, R₈₀, R₈₁, R₉₀, R₉₁ peuvent être protégés à l'aide de groupements protecteurs classiques et déprotégés au moment opportun, que les groupements hydroxy présents en ces mêmes positions peuvent être oxydés par des méthodes classiques de la chimie, en groupement oxo, que inversement les groupements oxo présents en ces mêmes positions peuvent être réduits par des réducteurs classiques, à tout moment opportun de la synthèse, et que lorsque deux de ces groupements forment ensemble une liaison, cette dernière peut être introduite à tout moment jugé utile par l'homme de métier afin de faciliter la synthèse,
composés de formule (I) :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

15. Procédé de préparation selon la revendication 14 **caractérisé en en ce que** l'on utilise un substrat de formule (II') : 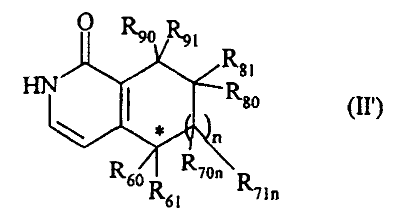 dans laquelle n, R₆₀, R₆₁, R₇₀ₙ, R₇₁ₙ, R₈₀, R₈₁, R₉₀ et R₉₁ sont tels que définis dans la formule (I) et * indique que le carbone portant les groupements R₆₀ et R₆₁ possède une configuration fixée (R) ou (S),
afin d'obtenir un composé de formule (I'): 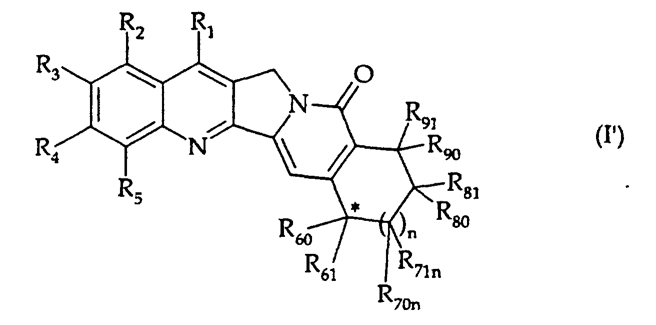 dans laquelle R₁, R₂, R₃, R₄, R₅, n, R₆₀, R₆₁, R₇₀ₙ, R₇₁ₙ, R₈₀, R₈₁, R₉₀ et R₉₁ sont tels que définis dans la formule (I) et * indique que la configuration du carbone est fixée.

16. Compositions pharmaceutiques contenant comme principe actif au moins un composé selon l'une quelconque des revendications 1 à 13, seul ou en combinaison avec un ou plusieurs excipients ou véhicules inertes, non toxiques, pharmaceutiquement acceptables.

17. Compositions pharmaceutiques selon la revendication 16 contenant au moins un principe actif selon l'une quelconque des revendications 1 à 13 utiles pour la fabrication de médicaments utiles dans le traitement des maladies cancéreuses.

## Patentansprüche

1. Verbindungen der Formel (I): 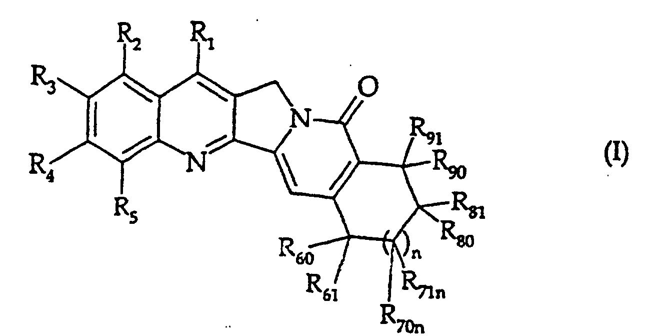 in der:
• n 0, 1 oder 2 bedeutet,
• R₁, R₂, R₃, R₄ und R₅ unabhängig voneinander ausgewählt sind aus einem Wasserstoffatom, Halogenatom, einer Alkylgruppe, Alkenylgruppe, Alkinylgruppe, Perhalogenalkylgruppe, (C₃-C₁₁)-Cycloalkylgruppe, (C₃-C₁₁)-Cycloalkylalkylgruppe. Hydroxygruppe, Hydroxyalkylgruppe, Alkoxygruppe, Alkoxyalkylgruppe, Alkoxycarbonylgruppe, Acyloxygruppe, Carboxygruppe, Nitrogruppe, Cyanogruppe, (gegebenenfalls am Stickstoffatom durch ein oder zwei Alkylgruppen substituierte) Aminocarbonylgruppe und Gruppen (CH₂)ₚ-NRₐR_{b} und -O-C(O)-N-RₐR_{b}, worin p eine ganze Zahl mit einem Wert zwischen 0 und 6 darstellt und Rₐ und R_{b} unabhängig voneinander ein Wasserstoffatom, eine Alkylgruppe, (C₃-C₁₁)-Cycloalkylgruppe, (C₃-C₁₁)-Cycloalkylalkylgruppe, Acylgruppe, gegebenenfalls substituierte Arylgruppe oder gegebenenfalls substituierte Arylalkylgruppe bedeuten oder Rₐ und R_{b} gemeinsam mit dem sie tragenden Stickstoffatom eine Pyrrolylgruppe, Piperidinylgruppe oder Piperazinylgruppe bilden, wobei jede dieser cyclischen Gruppen gegebenenfalls substituiert sein kann.
oder zwei benachbarte Gruppen R₂, R₃, R₄ und R₅ zusammen mit den sie tragenden Kohlenstoffatomen eine Gruppe -O-(CH₂)ₜ-O bilden, worin t eine ganze Zahl zwischen 1 und 3 einschließlich bedeutet,
• R₆₀, R₇₀ₙ, R₈₀ und R₉₀ unabhängig von einander ein Wasserstoffatom, eine Hydroxygruppe, Alkoxygruppe oder eine Gruppe 0-(CO)-X oder O-(CO)-NXW bedeuten, worin X und W unabhängig von einander eine Alkylgruppe, Alkenylgruppe, Alkinylgruppe, (C₃-C₁₁)-Cycloalkylgruppe, (C₃-C₁₁)-Cycloalkylalkylgruppe, gegebenenfalls substituierte Arylgruppe oder gegebenenfalls substituierte Arylalkylgruppe bedeuten,
• R₆₁, R₇₁ₙ, R₈₁ und R₉₁ unabhängig von einander ein Wasserstoffatom, eine Alkylgruppe, Alkenylgruppe, Alkinylgruppe, oder jeweils zwei dieser Gruppen an benachbarten Kohlenstoffatomen gemeinsam eine Bindung bilden oder eine Oxirangruppe oder zwei geminale Gruppen (R₆₀ und R₆₁) und/oder (R₇₀ₙ und R₇₁ₙ) und/oder (R₈₀ und R₈₁) und/oder (R₉₀ und R₉₁) gemeinsam eine Oxogruppe oder eine Gruppe -O-(CH₂)ₜ₁-O bilden, wobei t₁ eine ganze Zahl mit einem Wert zwischen 1 und 3 einschließlich darstellt,
mit der Maßgabe, daß nicht sämtliche Gruppen R₆₀, R₆₁, R₇₀ₙ, R₇₁ₙ, R₈₀, R₈₁, R₉₀ und R₉₁ Wasserstoffatome bedeuten,
deren Enantiomere, Diastereoisomere sowie deren Additionssalze mit einer pharmazeutisch annehmbaren Säure oder Base,
mit der Maßgabe, daß:
- der Begriff Alkyl für eine geradkettige oder verzweigte Kette mit 1 bis 6 Kohlenstoffatomen steht,
- der Begriff Alkenyl für eine geradkettige oder verzweigte Kette mit 2 bis 6 Kohlenstoffatomen steht, die 1 bis 3 Doppelbildungen enthält,
- der Begriff Alkinyl für eine geradkettige oder verzweigte Kette mit 2 bis 6 Kohlenstoffatomen steht, die 1 bis 3 Dreifachbindungen enthält,
- der Begriff Alkoxy für eine geradkettige oder verzweigte Alkyl-oxy-Gruppe steht, die 1 bis 6 Kohlenstoffatome enthält,
- der Begriff Acyl für eine geradkettige oder verzweigte Alkyl-carbonyl-Gruppe steht, die 1 bis 6 Kohlenstoffatome enthält,
- der Begriff Aryl für eine Phenyl- oder Naphthyl-Gruppe steht,
- der Begriff "substituiert" wenn er die Aryl- oder Arylalkyl-Gruppen betrifft bedeutet, daß die betreffenden Gruppen durch ein oder mehrere Halogenatome und/oder Alkylgruppen, Alkoxygruppen, Hydroxygruppen, Cyanogruppen. Nitrogruppen und/oder (gegebenenfalls durch ein oder zwei Alkylgruppen substituierte) Aminogruppen substituiert sind,
- der Begriff "substituiert" wenn er die Pyrrolyl-, Piperidinyl- oder Piperazinyl-Gruppen betrifft, bedeutet, daß die betreffenden Gruppen durch ein oder mehrere Alkylgruppen Alkoxygruppen, Arylgruppen. Arylalkylgruppen. Aryloxygruppen und/oder Aryloxyalkylgruppen substituiert sind.

2. Verbindungen der Formel (I) nach Anspruch 1, worin n den Wert 0 besitzt, deren Enantiomere, Diastereoisomere sowie deren Additionssalze mit einer pharmazeutisch annehmbaren Säure oder Base.

3. Verbindungen der Formel (I) nach Anspruch 1, worin n den Wert 1 besitzt, deren Enantiomere, Diastereoisomere sowie deren Additionssalze mit einer pharmazeutisch annehmbaren Säure oder Base.

4. Verbindungen der Formel (I) nach Anspruch 1, worin n den Wert 2 besitzt, deren Enantiomere, Diastereoisomere sowie deren Additionssalze mit einer pharmazeutisch annehmbaren Säure oder Base.

5. Verbindungen der Formel (I) nach Anspruch 1, worin R₆₀ eine Hydroxygruppe und R₆₁ eine Alkylgruppe bedeuten, deren Enantiomere, Diastereoisomere sowie deren Additionssalze mit einer pharmazeutisch annehmbaren Säure oder Base.

6. Verbindungen der Formel (I) nach Anspruch 1, worin R₈₀ und R₈₁ gemeinsam eine Oxogruppe bilden, deren Enantiomere, Diastereoisomere sowie deren Additionssalze mit einer pharmazeutisch annehmbaren Säure oder Base.

7. Verbindungen der Formel (I) nach Anspruch 1, worin R₉₀ und R₉₁ gemeinsam eine Oxogruppe bilden, deren Enantiomere, Diastereoisomere sowie deren Additionssalze mit einer pharmazeutisch annehmbaren Säure oder Base.

8. Verbindungen der Formel (I) nach Anspruch 1, worin R₈₀ und R₈₁ sowie R₉₀ und R₉₁ zwei Oxogruppen bilden, deren Enantiomere, Diastereoisomere sowie deren Additionssalze mit einer pharmazeutisch annehmbaren Säure oder Base.

9. Verbindungen der Formel (I) nach Anspruch 1, worin R₁ ein Wasserstoffatom bedeutet, deren Enantiomere, Diastereoisomere sowie deren Additionssalze mit einer pharmazeutisch annehmbaren Säure oder Base.

10. Verbindungen der Formel (I) nach Anspruch 1, worin R₂, R₃, R₄ und R₅ aus Wasserstoffatomen, Halogenatomen, Alkylgruppen oder Alkoxygruppen ausgewählt sind oder zwei dieser Gruppen, wenn sie an zwei benachbarte Kohlenstoffatome gebunden sind, gemeinsam eine Methylendioxy- oder Ethylendioxy-Gruppe bilden, deren Enantiomere, Diastereoisomere sowie deren Additionssalze mit einer pharmazeutisch annehmbaren Säure oder Base.

11. Verbindungen der Formel (I) nach Anspruch 1, worin R₁, R₂ und R₅ jeweils ein Wasserstoffatom bedeuten, R₃ und R₄ aus Wasserstoffatomen, Halogenatomen, Alkylgruppen und Alkoxygruppen ausgewählt sind oder gemeinsam eine Methylendioxygruppe bilden, R₆₀, R₇₀ₙ, R₈₀ und R₉₀ unabhängig von einander Wasserstoffatome, Hydroxygruppen oder Alkoxygruppen bedeuten und R₆₁, R₇₁ₙ, R₈₁ und R₉₁ unabhängig von einander Wasserstoffatome oder Alkylgruppen bedeuten, oder jeweils zwei dieser Gruppen an benachbarten Kohlenstoffatomen gemeinsam eine Bindung oder eine Oxirangruppe bilden, oder zwei geminale Gruppen (R₆₀ und R₆₁) und/oder (R₇₀ₙ und R₇₁ₙ) und/oder (R₈₀ und R₈₁) und/oder (R₉₀ und R₉₁) gemeinsam eine Oxogruppe bilden, deren Enantiomere, Diastereoisomere sowie deren Additionssalze mit einer pharmazeutisch annehmbaren Säure oder Base.

12. Verbindung der Formel (I) nach Anspruch 1, nämlich:
- 3-Chlor-7-ethyl-7-hydroxy-2-methyl-9,12-dihydro-7*H*-cyclopenta[6,7]indolizino[1,2-b]chinolin-8,10-on.

13. Verbindung der Formel (I) nach Anspruch 1, nämlich:
- 2,3-Difluor-7-ethyl-7-hydroxy-9,12-dihydro-7*H*-cyclopenta[6,7] indolizino[1,2-b]chinolin-8,10-dion.

14. Verfahren zur Herstellung der Verbindungen der Formel (I) nach Anspruch 1, **dadurch gekennzeichnet, daß** man als Ausgangsprodukt eine Verbindung der Formel (II) verwendet: 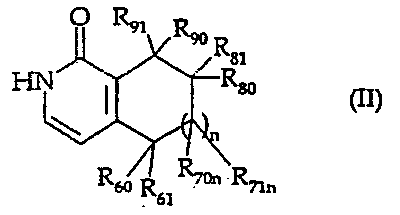 in der n, R₆₀, R₆₁, R₇₀ₙ, R₇₁ₙ, R₈₀, R₈₁, R₉₀ und R₉₁ die bezüglich der Formel (I) angegebenen Bedeutungen besitzen,
welche man in basischem Medium mit einer Verbindung der Formel (III) kondesiert: 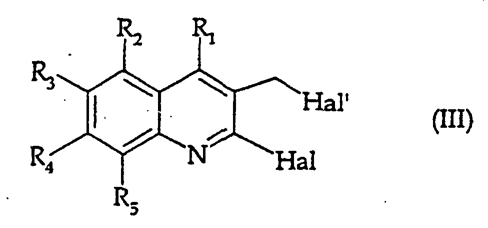 in der R₁, R₂, R₃, R₄ und R₅ die bezüglich der Formel (I) angegebenen Bedeutungen besitzen und Hal und Hal' unabhängig von einander ein Wasserstoffatom bedeuten,
oder mit einer Verbindung der Formel (III') kondensiert: 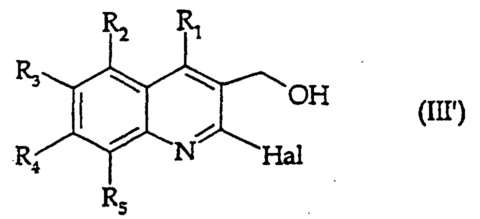 in der R₁, R₂, R₃, R₄, R₅ und Hal die oben angegebenen Bedeutungen besitzen.
zur Bildung einer Verbindung der Formel (IV): 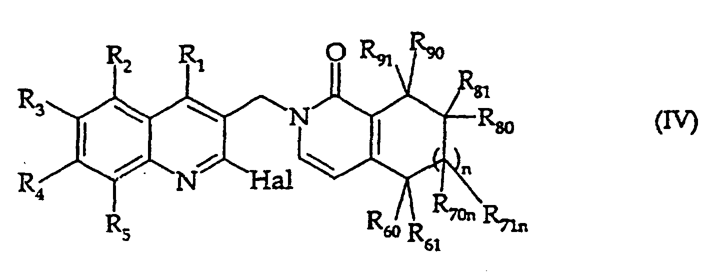 in der R₁, R₂, R₃, R₄, R₅, n, R₆₀, R₆₁, R₇₀ₙ, R₇₁ₙ, R₈₀, R₈₁, R₉₀, R₉₁ und Hal die oben angegebenen Bedeutungen besitzen,
welche Verbindung (IV) man einer durch ein Palladiumderivat katalysierten intramolekularen Cyclisierungsreaktion unterwirft zur Bildung der Verbindung der Formel (I),
wobei es sich versteht, daß mit dem Ziel das obige Verfahren zu vereinfachen die in R₆₀, R₆₁, R₇₀ₙ, R₇₁ₙ, R₈₀, R₈₁, R₉₀ und R₉₁ vorhandenen reaktlven Gruppen. mit Hilfe von klassischen Schutzgruppen geschützt und in einem geeigneten Moment wieder von den Schutzgruppen befreit werden können, daß in Jedem geeigneten Augenblick der Synthese die in den gleichen Positionen vorhandenen Hydroxygruppen mit Hilfe klassischer Methoden der Chemie zu Oxogruppen oxidiert werden können und umgekehrt die in diesen gleichen Positionen vorhandenen Oxogruppen mit Hilfe klassischer Reduktionsmittel reduziert werden können und daß wenn diese beiden Gruppen gemeinsam eine Bindung bilden, diese letztere in jedem von dem Fachmann als nützlich angesehenen Zeitpunkt zur Erleichterung der Synthese eingeführt werden kann,
welche Verbindungen der Formel (I):
- gegebenenfalls mit Hilfe einer klassischen Reinigungsmethode gereinigt werden können,
- gegebenenfalls mit Hilfe einer klassischen Trennmethode in ihre Stereoisomeren getrennt werden können,
- und gewünschtenfalls in ihre Säureadditionssalze mit einer pharmazeutisch annehmbaren Säure oder Base umgewandelt werden können.

15. Verfahren nach Anspruch 14, **dadurch gekennzeichnet, daß** man ein Substrat der Formel (II') verwendet: 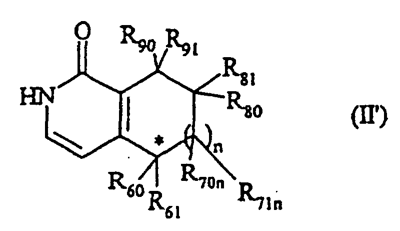 in der n, R₆₀, R₆₁, R₇₀ₙ, R₇₁ₙ, R₈₀, R₈₁, R₉₀ und R₉₁ die bezüglich der Formel (I) angegebenen Bedeutungen besitzen und * bedeutet, daß das die Gruppen R₆₀ und R₆₁ tragende Kohlenstoffatom eine feste Konfiguration (R) oder (S) aufweist,
zur Bildung einer Verbindung der Formel (I'): 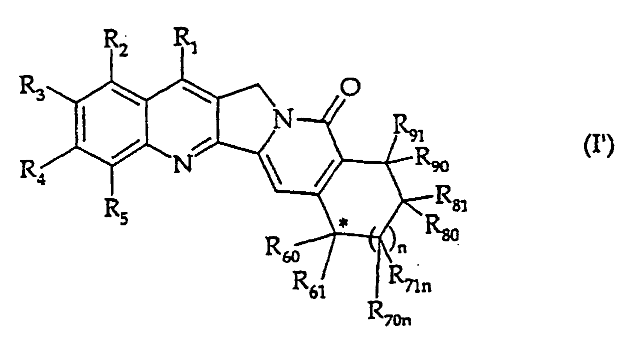 in der R₁, R₂, R₃, R₄, R₅, n, R₆₀, R₆₁, R₇₀ₙ, R₇₁ₙ, R₈₀, R₈₁, R₉₀ und R₉₁ die bezüglich der Formel (I) angegebenen Bedeutungen besitzen und * bedeutet, daß die Konfiguration des Kohlenstoffatoms definiert ist.

16. Pharmazeutische Zubereitungen enthaltend als Wirkstoff mindestens eine Verbindung nach einem Ansprüche 1 bis 13 allein oder in Kombination mit einem oder mehreren inerten, nichttoxischen, pharmazeutisch annehmbaren Trägermaterialen oder Bindemitteln.

17. Pharmazeutische Zubereitungen nach Anspruch 16, enthaltend mindestens einen Wirkstoff nach einem der Ansprüche 1 bis 13 für die Herstellung von Arzneimitteln zur Behandlung von Krebserkrankungen.

## Claims

1. Compounds of formula (I): 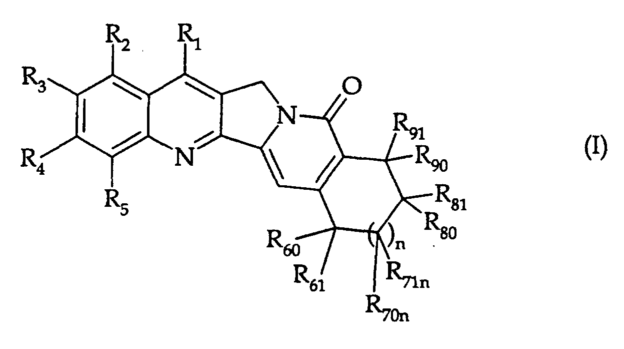 wherein :
• n is 0, 1 or 2,
• R₁, R₂, R₃, R₄ and R₅ are selected each independently of the others from a hydrogen atom, a halogen atom, an alkyl group, an alkenyl group, an alkynyl group, a perhaloalkyl group, a (C₃-C₁₁)cycloalkyl group, a (C₃-C₁₁)cycloalkyl-alkyl group, a hydroxy group, a hydroxyalkyl group, an alkoxy group, an alkoxyalkyl group, an alkoxycarbonyl group, an acyloxy group, a carboxy group, a nitro group, a cyano group, an aminocarbonyl group (optionally substituted on the nitrogen atom by one or two alkyl groups), and the groups (CH₂)ₚ-NRₐR_{b} and -O-C(O)-N-RₐR_{b}, wherein p is an integer from 0 to 6, and Rₐ and R_{b} each independently of the other represents a hydrogen atom, an alkyl group, a (C₃-C₁₁)cycloalkyl group, a (C₃-C₁₁)cycloalkyl-alkyl group, an acyl group, an optionally substituted aryl group or an optionally substituted arylalkyl group, or Ra and Rb form together with the nitrogen atom carrying them a pyrrolyl, piperidyl or piperazinyl group, it being possible for each of those cyclic groups to be optionally substituted,
or two adjacents groups R₂, R₃, R₄ and R₅ form together with the carbon atoms carrying them a group -O-(CH₂)ₜ-O, t being an integer from 1 to 3 inclusive,
• R₆₀, R₇₀ₙ, R₈₀ and R₉₀ each independently of the others represents a hydrogen atom, a hydroxy group, an alkoxy group, or a group O-(CO)-X or O-(CO)-NXW wherein X and W each independently of the other represents an alkyl group, an alkenyl group, an alkynyl group, a (C₃-C₁₁)cycloalkyl group, a (C₃-C₁₁)cycloalkyl-alkyl group, an optionally substituted aryl group or an optionally substituted arylalkyl group,
• R₆₁, R₇₁ₙ, R₈₁ and R₉₁ each independently of the others represents a hydrogen atom, an alkyl group, an alkenyl group or an alkynyl group, or, taken in pairs on adjacent carbon atoms, together form a bond or an oxirane group, or two geminal groups (R₆₀ and R₆₁) and/or (R₇₀ₙ and R₇₁ₙ) and/or (R₈₀ and R₈₁) and/or (R₉₀ and R₉₁) together form an oxo group or a group -O-(CH₂)ₜ₁-O, t₁ being an integer from 1 to 3 inclusive,
with the proviso that R₆₀, R₆₁, R₇₀ₙ, R₇₁ₙ, R₈₀, R₈₁, R₉₀ and R₉₁ do not all represent a hydrogen atom,
their enantiomers, diastereoisomers, and addition salts thereof with a pharmaceutically acceptable acid or base,
it being understood that :
- the term "alkyl" denotes a linear or branched chain having from 1 to 6 carbon atoms,
- the term "alkenyl" denotes a linear or branched chain having from 2 to 6 carbon atoms and containing from 1 to 3 double bonds,
- the term "alkynyl" denotes a linear or branched chain having from 2 to 6 carbon atoms and containing from 1 to 3 triple bonds,
- the term "alkoxy" denotes a linear or branched alkyl-oxy radical containing from 1 to 6 carbon atoms,
- the term "acyl" denotes a linear or branched alkyl-carbonyl radical containing from 1 to 6 carbon atoms,
- the term "aryl" represents a phenyl or naphthyl group,
- the expression "substituted" when used in relation to aryl or arylalkyl groups means that the groups in question are substituted by one or more halogen atoms and/or groups alkyl, alkoxy, hydroxy, cyano, nitro and/or amino (optionally substituted by one or two alkyl groups),
- the expression "substituted" when used in relation to pyrrolyl, piperidyl or piperazinyl groups means that the groups in question are substituted by one or more alkyl, alkoxy, aryl, arylalkyl, aryloxy and/or aryloxyalkyl groups.

2. Compounds of formula (I) according to claim 1, wherein n is 0, their enantiomers, diastereoisomers and addition salts thereof with a pharmaceutically acceptable acid or base.

3. Compounds of formula (I) according to claim 1 wherein n is 1, their enantiomers, diastereoisomers and addition salts thereof with a pharmaceutically acceptable acid or base.

4. Compounds of formula (I) according to claim 1 wherein n is 2, their enantiomers, diastereoisomers and addition salts thereof with a pharmaceutically acceptable acid or base.

5. Compounds of formula (I) according to claim 1 wherein R₆₀ represents a hydroxy group and R₆₁ represents an alkyl group, their enantiomers, diastereoisomers and addition salts thereof with a pharmaceutically acceptable acid or base.

6. Compounds of formula (I) according to claim 1 wherein R₈₀ and R₈₁ together form an oxo group, their enantiomers, diastereoisomers and addition salts thereof with a pharmaceutically acceptable acid or base.

7. Compounds of formula (I) according to claim 1 wherein R₉₀ and R₉₁ together form an oxo group, their enantiomers, diastereoisomers and addition salts thereof with a pharmaceutically acceptable acid or base.

8. Compounds of formula (I) according to claim 1 wherein R₈₀ with R₈₁ and R₉₀ with R₉₁ form two oxo groups, their enantiomers, diastereoisomers and addition salts thereof with a pharmaceutically acceptable acid or base.

9. Compounds of formula (I) according to claim 1 wherein R₁ represents a hydrogen atom, their enantiomers, diastereoisomers and addition salts thereof with a pharmaceutically acceptable acid or base.

10. Compounds of formula (I) according to claim 1 wherein R₂, R₃, R₄ and R₅ are selected from a hydrogen atom, a halogen atom, an alkyl group and an alkoxy group, or two of those groups, when bonded to two adjacent carbon atoms, together form a methylenedioxy or ethylenedioxy group, their enantiomers, diastereoisomers and addition salts thereof with a pharmaceutically acceptable acid or base.

11. Compounds of formula (I) according to claim 1 wherein each of R₁, R₂ and R₅ represents a hydrogen atom, R₃ and R₄ are selected from a hydrogen atom, a halogen atom, an alkyl group and an alkoxy group, or together form a methylenedioxy group, R₆₀, R₇₀ₙ, R₈₀ and R₉₀ each independently of the others represents a hydrogen atom, a hydroxy group or an alkoxy group, and R₆₁, R₇₁ₙ, R₈₁ and R₉₁ each independently of the others represents a hydrogen atom or an alkyl group, or, taken in pairs on adjacent carbon atoms, together form a bond or an oxirane group, or two geminal groups (R₆₀ and R₆₁) and/or (R₇₀ₙ and R₇₁ₙ) and/or (R₈₀ and R₈₁) and/or (R₉₀ and R₉₁) together form an oxo group, their enantiomers, diastereoisomers and addition salts thereof with a pharmaceutically acceptable acid or base.

12. Compound of formula (I) according to claim 1 which is :
- 3-chloro-7-ethyl-7-hydroxy-2-methyl-9,12-dihydro-7*H*-cyclopenta[6,7]-indolizino[1,2-b]quinolin-8,10-one

13. Compound of formula (I) according to claim 1 which is:
- 2,3-difluoro-7-ethyl-7-hydroxy-9,12-dihydro-7*H*-cyclopenta[6,7]indolizino-[1,2-b]quinoline-8,10-dione

14. Process for the preparation of compounds of formula (I) according to claim 1, **characterised in that** there is used as starting material a compound of formula (II): 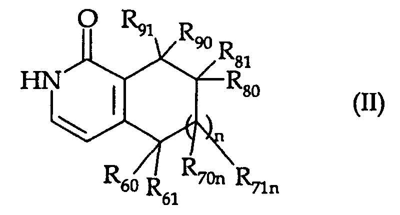 wherein n, R₆₀, R₆₁, R₇₀ₙ, R₇₁ₙ, R₈₀, R₈₁, R₉₀ and R₉₁ are as defined for formula (I),
which is condensed, in a basic medium, with a compound of formula (III): 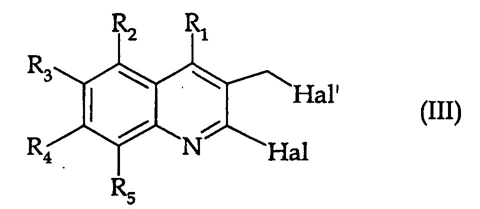 wherein R₁, R₂, R₃, R₄ and R₅ are as defined for formula (I), and Hal and Hal' each independently of the other represents a halogen atom,
or with a compound of formula (III'): 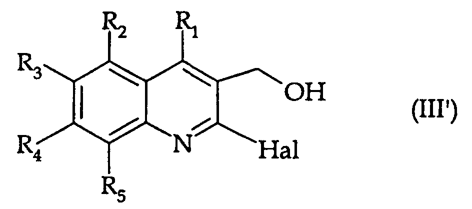 wherein R₁, R₂, R₃, R₄, R₅ and Hal are as defined hereinbefore,
to yield a compound of formula (IV) : 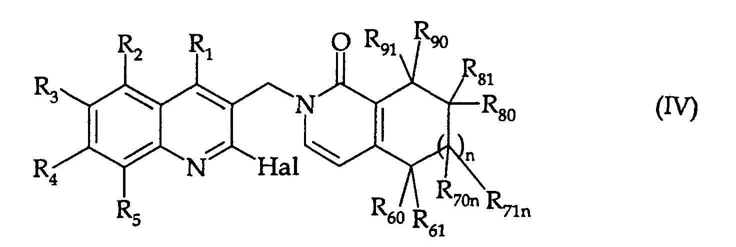 wherein R₁, R₂, R₃, R₄, R₅, n, R₆₀, R₆₁, R₇₀ₙ, R₇₁ₙ, R₈₀, R₈₁, R₉₀, R₉₁ and Hal are as defined hereinbefore,
which compound (IV) is subjected to an intramolecular cyclisation reaction catalysed by a palladium compound, to yield a compound of formula (I),
it being understood, for the purpose of simplifying the above process, that the reactive groups present in R₆₀, R₆₁, R₇₀ₙ, R₇₁ₙ, R₈₀, R₈₁, R₉₀ and R₉₁ can be protected by conventional protecting groups and deprotected at the appropriate time point, that the hydroxy groups present in those same positions can be oxidised by conventional chemistry methods to oxo groups, and, conversely, the oxo groups present in those same positions can be reduced by conventional reducing agents at any appropriate time point in the synthesis, and that, when two of those groups together form a bond, the latter can be introduced at any time point deemed suitable by the person skilled in the art in order to facilitate the synthesis,
which compounds of formula (I) :
- can be purified, if necessary, according to a conventional purification technique,
- are separated, where appropriate, into their stereoisomers according to a conventional separation technique,
- are converted, if desired, into addition salts thereof with a pharmaceutically acceptable acid or base.

15. Preparation process according to claim 14, **characterised in that** there is used a substrate of formula (II') : 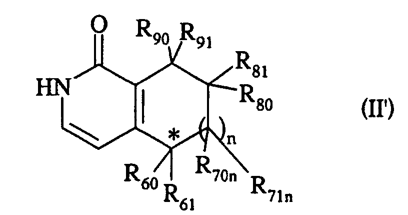 wherein n, R₆₀, R₆₁, R₇₀ₙ, R₇₁ₙ, R₈₀, R₈₁, R₉₀ and R₉₁ are as defined for formula (I) and * indicates that the carbon atom carrying the groups R₆₀ and R₆₁ has a fixed configuration (R) or (S),
in order to obtain a compound of formula (I') : 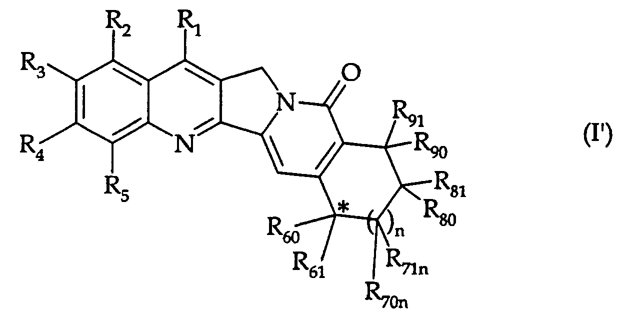 wherein R₁, R₂, R₃, R₄, R₅, n, R₆₀, R₆₁, R₇₀ₙ, R₇₁ₙ, R₈₀, R₈₁, R₉₀ and R₉₁ are as defined for formula (I) and * indicates that the configuration of the carbon atom is fixed.

16. Pharmaceutical compositions comprising as active ingredient at least one compound according to any one of claims 1 to 13, alone or in combination with one or more inert, non-toxic pharmaceutically acceptable excipients or carriers.

17. Pharmaceutical compositions according to claim 16 comprising at least one active ingredient according to any one of claims 1 to 13 for use in the production of medicaments for use in the treatment of cancerous diseases.
